# EUROPEAN PATENT SPECIFICATION

(11) **EP 1 415 002 B1**
(45) Date of publication and mention of the grant of the patent: **02.02.2005**
(21) Application number: 02788114.3
(22) Date of filing: 16.12.2002
(51) Int. Cl.: C12Q 1/68

(54) **FOCUSSED ANTIBODY TECHNOLOGY**
GEZIELTES ANTIKÖRPER-VERFAHREN
TECHNOLOGIE D'ANTICORPS CIBLEE

(30) Priority: 19.12.2001 GB 0130267
(43) Date of publication of application: 06.05.2004
(73) Proprietor: Neutec Pharma PLC, Manchester, Lancashire M13 9WL (GB)
(72) Inventor: BURNIE, James, Peter, Alderley Edge, Cheshire SK9 7PY (GB); MATTHEWS, Ruth, Christine, Alderley Edge, Cheshire SK9 7PY (GB); RIGG, Gordon, Patrick, Glossop, Derbyshire SK13 1DP (GB); WILLIAMSON, Peter, Manchester, Lancashire M21 7LT (GB)
(74) Representative: Robertson, James Alexander
(86) International application number: PCT/GB2002/005690
(87) International publication number: WO 2003/052416

(56) References cited:
- EP-A- 0 376 851
- WO-A-02/055559
- CURRIER, J. R. ET AL: "Mitogens, superantigens and nominal antigens elicit distinctive patters of TCRB CDR3 diversity" HUMAN IMMUNOLOGY, vol. 48, June 1996 (1996-06), pages 39-51, XP000647838 USA
- RINALDI MONICA ET AL: "Antibodies elicited by naked DNA vaccination against the complementary-determining region 3 hypervariable region of immunoglobulin heavy chain idiotypic determinants of B-lymphoproliferative disorders specifically react with patients' tumor cells." CANCER RESEARCH, vol. 61, no. 4, 15 February 2001 (2001-02-15), pages 1555-1562, XP001153568 ISSN: 0008-5472
- WEN Y-J ET AL: "IN-VIVO IMMUNE RESPONSES TO IDIOTYPIC VH COMPLEMENTARITY-DETERMINING REGION 3 PEPTIDE VACCINATION IN B-CELL NON-HODGKIN'S LYMPHOMA" BRITISH JOURNAL OF HAEMATOLOGY, OXFORD, GB, vol. 103, no. 3, December 1998 (1998-12), pages 663-668, XP001005275 ISSN: 0007-1048
- RINALDI M ET AL: "STRATEGIES TO ELICIT ANTI-IDIOTYPIC IMMUNE RESPONSE IN B-LYMPHOMA PATIENTS PEPTIDE AND GENETIC IMMUNIZATION" ADVANCES IN EXPERIMENTAL MEDICINE AND BIOLOGY, SPRING ST., NY, US, vol. 451, 1998, pages 323-330, XP001005261 ISSN: 0065-2598

### SEQUENCE LISTING

<110> NeuTec Pharma PLC
<120> Focussed Antibody Technology
<130> N01/0731/PCT
<150> GB 0130267.8
   <151> 2001-12-19
<160> 72
<170> PatentIn version 3.1
<210> 1
   <211> 24
   <212> DNA
   <213> Artificial Sequence
<220>
   <223> PCR Primer
<400> 1
   gtccaccttg gtgttgctgg gctt 24
<210> 2
   <211> 24
   <212> DNA
   <213> Artificial Sequence
<220>
   <223> PCR Primer
<400> 2
   agactctccc ctgttgaagc tctt 24
<210> 3
   <211> 24
   <212> DNA
   <213> Artificial Sequence
<220>
   <223> PCR Primer
<400> 3
   tgaggagacg gtgaccaggg tgcc 24
<210> 4
   <211> 24
   <212> DNA
   <213> Artificial Sequence
<220>
   <223> PCR Primer
<400> 4
   tgaagagacg gtgaccattg tccc 24
<210> 5
   <211> 24
   <212> DNA
   <213> Artificial Sequence
<220>
   <223> PCR Primer
<400> 5
   tgaggagacg gtgaccaggg ttcc 24
<210> 6
   <211> 24
   <212> DNA
   <213> Artificial Sequence
<220>
   <223> PCR Primer
<400> 6
   tgaggagacg gtgaccgtgg tccc 24
<210> 7
   <211> 23
   <212> DNA
   <213> Artificial Sequence
<220>
   <223> PCR Primer
<400> 7
   caggtgcagc tggtgcagtc tgg 23
<210> 8
   <211> 23
   <212> DNA
   <213> Artificial Sequence
<220>
   <223> PCR Primer
<400> 8
   caggtgaact taagggagtc tgg 23
<210> 9
   <211> 23
   <212> DNA
   <213> Artificial Sequence
<220>
   <223> PCR Primer
<400> 9
   caggtgcagc tggtggagtc tgg 23
<210> 10
   <211> 23
   <212> DNA
   <213> Artificial Sequence
<220>
   <223> PCR Primer
<400> 10
   caggtgcagc tgcaggagtc ggg 23
<210> 11
   <211> 23
   <212> DNA
   <213> Artificial Sequence
<220>
   <223> PCR Primer
<400> 11
   caggtgcagc tacagcagtg ggg 23
<210> 12
   <211> 23
   <212> DNA
   <213> Artificial Sequence
<220>
   <223> PCR Primer
<400> 12
   gaggtgcagc tgttgcagtc tgc 23
<210> 13
   <211> 23
   <212> DNA
   <213> Artificial Sequence
<220>
   <223> PCR Primer
<400> 13
   caggtacagc tgcagcagtc agg 23
<210> 14
   <211> 24
   <212> DNA
   <213> Artificial Sequence
<220>
   <223> PCR Primer
<400> 14
   acgtttgatt tccaccttgg tccc 24
<210> 15
   <211> 24
   <212> DNA
   <213> Artificial Sequence
<220>
   <223> PCR Primer
<400> 15
   acgtttgatc tccagcttgg tccc 24
<210> 16
   <211> 24
   <212> DNA
   <213> Artificial Sequence
<220>
   <223> PCR Primer
<400> 16
   acgtttgata tccactttgg tccc 24
<210> 17
   <211> 24
   <212> DNA
   <213> Artificial Sequence
<220>
   <223> PCR Primer
<400> 17
   acgtttgatc tccaccttgg tccc 24
<210> 18
   <211> 24
   <212> DNA
   <213> Artificial Sequence
<220>
   <223> PCR Primer
<400> 18
   acgtttattc tccagtcgtg tccc 24
<210> 19
   <211> 23
   <212> DNA
   <213> Artificial Sequence
<220>
   <223> PCR Primer
<400> 19
   gacatccaga tgacccagtc tcc 23
<210> 20
   <211> 23
   <212> DNA
   <213> Artificial Sequence
<220>
   <223> PCR Primer
<400> 20
   gatgttgtga tgactcagtc tcc 23
<210> 21
   <211> 23
   <212> DNA
   <213> Artificial Sequence
<220>
   <223> PCR Primer
<400> 21
   gaaattgtgt tgacgcagtc tcc 23
<210> 22
   <211> 23
   <212> DNA
   <213> Artificial Sequence
<220>
   <223> PCR Primer
<400> 22
   gacatcgtga tgacccagtc tcc 23
<210> 23
   <211> 23
   <212> DNA
   <213> Artificial Sequence
<220>
<223> PCR Primer
<210> 24
   <211> 23
   <212> DNA
   <213> Artificial Sequence
<220>
   <223> PCR primer
<400> 24
   gaaattgtgc tgactcagtc tcc 23
<210> 25
   <211> 17
   <212> DNA
   <213> Artificial Sequence
<220>
   <223> PCR Primer
<400> 25
   gtaaaacgac ggccagt 17
<210> 26
   <211> 16
   <212> DNA
   <213> Artificial Sequence
<220>
   <223> PCR Primer
<400> 26
   aacagctatg accatg 16
<210> 27
   <211> 7
   <212> PRT
   <213> Homo sapiens
<400> 27
   Arg Asn Asn Trp Pro Pro Thr
   1 5
<210> 28
   <211> 11
   <212> PRT
   <213> Homo sapiens
<400> 28
   Arg Ser Gly Ser His Tyr Asp Ala Phe Asn Ile
   1 5 10
<210> 29
   <211> 7
   <212> PRT
   <213> Homo sapiens
<400> 29
   Val Thr Gly Cys Phe Asp His
   1 5
<210> 30
   <211> 10
   <212> PRT
   <213> Homo sapiens
<400> 30
   Leu His Gly Phe Gly His Gly Phe Arg Ile
   1 5 10
<210> 31
   <211> 6
   <212> PRT
   <213> Homo sapiens
<400> 31
   Gly Glu Ile Phe Asp Tyr
   1 5
<210> 32
   <211> 12
   <212> PRT
   <213> Homo sapiens
<400> 32
   Leu Leu Gly Gly Ser Ser Gly Arg Tyr Met Asp Val
   1 5 10
<210> 33
   <211> 17
   <212> PRT
   <213> Homo sapiens
<400> 33
   His Arg Gly Gly Pro Gln Cys His Asn Leu Asn Cys Tyr Thr Leu Asp
   1 5 10 15
   Phe
<210> 34
   <211> 13
   <212> PRT
   <213> Homo sapiens
<400> 34
   Glu Ala Glu Ser Tyr Ala Glu Arg Ile Gly Phe Asp Tyr
   1 5 10
<210> 35
   <211> 13
   <212> PRT
   <213> Homo sapiens
<400> 35
   Tyr Asp Val Val Val Thr Gly Lys Tyr Ala Phe Asp Ile
   1 5 10
<210> 36
   <211> 14
   <212> PRT
   <213> Homo sapiens
<400> 36
   Arg Arg Tyr His Asp Leu Thr Thr Asn Asn Trp Phe Asp Pro
   1 5 10
<210> 37
   <211> 16
   <212> PRT
   <213> Homo sapiens
<400> 37
   Asn Val Gly Ser Gly Ser Tyr Tyr Thr Gly Gly His Trp Phe Asp Pro
   1 5 10 15
<210> 38
   <211> 17
   <212> PRT
   <213> Homo sapiens
<400> 38
   His Arg Gly Gly Pro Gln Cys His Asn Leu Asn Cys Tyr Thr Leu Asp
   1 5 10 15
   Phe
<210> 39
   <211> 13
   <212> PRT
   <213> Homo sapiens
<400> 39
   Ala Arg Arg Tyr Gly Thr Ser Asn Tyr Cys Phe Asp His
   1 5 10
<210> 40
   <211> 15
   <212> PRT
   <213> Homo sapiens
<400> 40
   Asp Gly Leu Asp Ser Ala Tyr Cys Gly Gly Asp Cys Phe Gly Tyr
   1 5 10 15
<210> 41
   <211> 9
   <212> PRT
   <213> Homo sapiens
<400> 41
   Asp Gln Asp Ala Ala Pro Tyr Asp His
   1 5
<210> 42
   <211> 13
   <212> PRT
   <213> Homo sapiens
<400> 42
   Asp Leu Asp Gln Phe Tyr Tyr Tyr Tyr Gly Met Asp Val
   1 5 10
<210> 43
   <211> 13
   <212> PRT
   <213> Homo sapiens
<400> 43
   Glu Tyr Ser Ser Thr Trp Tyr Gly Ser Trp Phe Asp Pro
   1 5 10
<210> 44
   <211> 16
   <212> PRT
   <213> Homo sapiens
<400> 44
   Gly Asp Thr Ser Gly Tyr Tyr Pro Thr Ser Phe His Trp Phe Asp Pro
   1 5 10 15
<210> 45
   <211> 12
   <212> PRT
   <213> Homo sapiens
<400> 45
   Asp Arg Ser Gly Ser His Tyr Asp Ala Phe Asn Ile
   1 5 10
<210> 46
   <211> 12
   <212> PRT
   <213> Homo sapiens
<400> 46
   Leu Leu Gly Gly Ser Ser Gly Arg Tyr Met Asp Val
   1 5 10
<210> 47
   <211> 13
   <212> PRT
   <213> Homo sapiens
<400> 47
   Tyr Asp Val Val Val Thr Gly Lys Tyr Ala Phe Asp Ile
   1 5 10
<210> 48
   <211> 17
   <212> PRT
   <213> Homo sapiens
<400> 48
   His Arg Gly Gly Pro Gln Cys His Asn Leu Asn Cys Tyr Thr Leu Asp
   1 5 10 15
   Phe
<210> 49
   <211> 13
   <212> PRT
   <213> Homo sapiens
<400> 49
   Glu Ala Glu Ser Tyr Ala Glu Arg Ile Gly Phe Asp Tyr
   1 5 10
<210> 50
   <211> 16
   <212> PRT
   <213> Homo sapiens
<400> 50
   Asn Val Gly Ser Gly Ser Tyr Tyr Thr Gly Gly His Trp Phe Asp Pro
   1 5 10 15
<210> 51
   <211> 11
   <212> PRT
   <213> Homo sapiens
<400> 51
   Val Ala Thr Thr Thr Glu Trp Phe Leu Asp Phe
   1 5 10
<210> 52
   <211> 24
   <212> PRT
   <213> Homo sapiens
<400> 52
   Asp Gly Gly Arg Asp Pro Gly Asp Thr Thr Arg Val Ala Gly Ala Gln
   1 5 10 15
   His Leu Tyr Tyr Tyr Leu Asp Val
   20
<210> 53
   <211> 14
   <212> PRT
   <213> Homo sapiens
<400> 53
   Gly Trp His Glu Val Gly Ile Gly Tyr Pro Leu Phe Gln Asn
   1 5 10
<210> 54
   <211> 14
   <212> PRT
   <213> Homo sapiens
<400> 54
   Lys Asp Ser Ala Asp Gly Ser Met Asn Asp Tyr Phe Asp Ala
   1 5 10
<210> 55
   <211> 10
   <212> PRT
   <213> Homo sapiens
<400> 55
   Asp Cys Gly Gln Gly Gly Ser Arg Asp Ser
   1 5 10
<210> 56
   <211> 9
   <212> PRT
   <213> Homo sapiens
<400> 56
   Asp Gln Ser His Ala Ala Gln Gly Phe
   1 5
<210> 57
   <211> 18
   <212> PRT
   <213> Homo sapiens
<400> 57
Asp Phe
<210> 58
   <211> 11
   <212> PRT
   <213> Homo sapiens
<400> 58
   Thr Glu Met Ala Gly Asp Phe Asp Ser Asp Tyr
   1 5 10
<210> 59
   <211> 11
   <212> PRT
   <213> Homo sapiens
<400> 59
   Asp Arg Asp Trp Arg Glu Gly Gly Leu Asp Ser
   1 5 10
<210> 60
   <211> 11
   <212> PRT
   <213> Homo sapiens
<400> 60
   Glu Ala Leu Ala Gly Ile His Leu Tyr Asp His
   1 5 10
<210> 61
   <211> 11
   <212> PRT
   <213> Homo sapiens
<400> 61
   Gly Met Leu Arg Gly Leu Tyr Trp Phe Asp Pro
   1 5 10
<210> 62
   <211> 7
   <212> PRT
   <213> Homo sapiens
<400> 62
   Gly Arg Gly Tyr Ser Asp Tyr
   1 5
<210> 63
<211> 10
   <212> PRT
   <213> Homo sapiens
<400> 63
   Leu Pro Ala Thr Ala Ile Tyr Pro Leu Asp
   1 5 10
<210> 64
   <211> 15
   <212> PRT
   <213> Homo sapiens
<400> 64
   Pro Arg Gly Met Ile Arg Gly Val Leu Pro Asp Ala Phe Asp Ile
   1 5 10 15
<210> 65
   <211> 13
   <212> PRT
   <213> Homo sapiens
<400> 65
   Pro Ser Asp Gly Asp Tyr Gly Asp Ser Arg Val Asp Val
   1 5 10
<210> 66
   <211> 9
   <212> PRT
   <213> Homo sapiens
<400> 66
   Arg Arg Ser Glu Pro Ala Phe Asp Tyr
   1 5
<210> 67
   <211> 13
   <212> PRT
   <213> Homo sapiens
<400> 67
   Thr Arg Tyr Asn Thr Gly Leu Gly His Ala Ala Leu Asp
   1 5 10
<210> 68
   <211> 15
   <212> PRT
   <213> Homo sapiens
<400> 68
   Val Arg Tyr Phe Asp Trp Pro Arg Gly Asp Asn Trp Phe Asp Pro
   1 5 10 15
<210> 69
   <211> 4
   <212> PRT
   <213> Artificial Sequence
<220>
   <223> Used for defining CDR region
<220>
   <221> MISC_FEATURE
   <222> (3).. (3)
   <223> Any amino acid
<400> 69
   Phe Gly Xaa Gly
   1
<210> 70
   <211> 4
   <212> PRT
   <213> Artificial Sequence
<220>
   <223> Used for defining CDR region
<220>
   <221> MISC_FEATURE
   <222> (2)..(4)
   <223> Any amino acid
<400> 70
   Cys Xaa Xaa Xaa
   1
<210> 71
   <211> 5
   <212> PRT
   <213> Artificial Sequence
<220>
   <223> Used for defining CDR region
<400> 71
   Leu Glu Trp Ile Gly
   1 5
<210> 72
   <211> 4
   <212> PRT
   <213> Artificial Sequence
<220>
   <223> Used for defining CDR region
<220>
   <221> MISC_FEATORE
   <222> (3)..(3)
   <223> Any amino acid
<400> 72
   Trp Gly Xaa Gly
   1

## Description

The present invention is concerned with methods of identifying antibody specific for and which confer immunity against infection by micro-organisms such as bacteria, viruses and yeasts. Also provided are methods of manufacture of medicaments, methods of treatment of patients producing databases, databases produced by same, methods of preparing reports regarding same, methods of determining the efficacy of vaccines, databases produced by same, and methods of preparing reports regarding same.

During the course of microbial infection various adaptive strategies are employed by the immune system. One such strategy, and arguably the most important, is the production of an antibody response. Antibodies capable of binding antigens displayed by the infectious agent are produced and bind to and allow killing of the microorganism through complement activation, recruitment of macrophage and through direct interaction with microbe itself. The therapeutic efficacy of antibodies capable of binding a given antigen varies and this is reflected in the fact that antibody production by the immune system matures during the course of an infection and becomes focussed in the case of a patient successfully fighting off an infection.

The antibody response is elicited by the B cell repertoire where individual B cells each produce structurally diverse antibody molecules. The actual size of this B cell/antibody repertoire is unknown, but it is estimated that the random clonal frequency of reactivity for a given antigen may be as high as 1 in 100,000 in cultured B cells (Nobrega A *et al.,* Eur J Immunol. 1998 Apr;28(4):1204-15; PMID: 9565360). During the course of infection, antibodies capable of binding the pathogen are selected for by changes in the B cell population resulting in key antibodies being produced in large numbers. The mechanisms for these changes include clonal expansion, isotype switching, and somatic mutation of immunoglobulin variable regions. B cells responsible for generating antibodies which are able to bind pathogen multiply, thus skewing the B cell repertoire and changing the proportions of B cells. For example, in a study of B cells isolated from the blood of individuals with immunity and antibodies against *Plasmodium falciparum* (the causative agent of malaria) the frequency of circulatory B cells producing pathogen-reactive antibodies was as high as 1 in 1,403 (no pathogen reactivity could be found in non-infected individuals; Migot F *et al.,* Clin Exp Immunol. 1995 Dec;102(3):529-34; PMID: 8536368). In HIV infection, a similar distortion in the circulatory B cell repertoire has been observed where the frequency of HIV specific antibody-producing B cells was found to be nine times higher in HIV+ individuals compared to HIV- subjects (Zubler *RH et al.,* Clin Exp Immunol. 1992 Jan;87(1):31-6; PMID: 1733635).

Obviously it is desirable to be able to effect treatment of infection in patients. This can be done by e.g. vaccination or by passive immunotherapy, as well as by the use of other therapeutically effective chemicals. Typically, vaccination is achieved by isolating and purifying an immunogen from the infecting pathogen and using it as the basis, either in a modified or unmodified form, for a vaccine which is administered to patients to stimulate antibody production.

Passive immunotherapy can be achieved by the administration of antibody to a patient and is particularly effective in treating immunocompromised patients who are unable to respond to vaccination, and to patients who need immediate therapy and cannot wait for vaccination to take effect. Passive immunotherapy is typically achieved by administering to a patient a monoclonal antibody specific to an immunogen produced by a pathogen. Thus in order to effect treatment of the patient, an immunogen must first be isolated and purified, administered to test animals, and cells producing antibody specific against the immunogen cloned. The range of antibodies produced by the clones can then be tested for their therapeutic efficacy and a single monoclonal antibody selected which is then administered to patients to effect passive immunotherapy.

Obviously all of these techniques are somewhat complex, inconvenient, expensive and time consuming. In general they require that an immunogen is isolated from the infecting pathogen and used to generate antibody. Simply isolating the immunogen can be extremely difficult and time consuming. In particular, carbohydrate antigens pose great problems - they are displayed by the likes of Group B Meningitidis e.g. *Neisseria meningitidis* and *Streptococcus agalactiae.* There are also complex non-linear epitopes (i.e. having secondary, tertiary and quaternary structural features) which cannot be synthesised *in vitro.* As such it is simply impossible to isolate and produce the antigen for use as e.g. a vaccine. In addition, some epitopes are only produced by pathogens *in vivo* and are not synthesised *in vitro.* Various host-specific expression systems are well known, for example in gonorrhea (causative organism *Neisseria gonorrhoeae)* specific antigens are expressed upon infection of a host - such antigens fall into the general class of cryptantigens. Furthermore, highly labile antigens also pose problems to prior art systems since during use they simply degrade and the epitope they display is lost With this large range of epitopes/antigens whose identification and/or *in vitro* use is of great difficulty or impossible the present invention provides a solution and allows the production and isolation of antibodies specific against them.

In addition, the prior art typically has to attempt to achieve an equivalent of affinity maturation of antibodies by first synthesising a set of candidate antibodies specific to an antigen, testing them for their binding characteristics and then modifying the sequences of the candidates in order to optimise the binding. A thorough attempt at affinity maturation (i.e. optimising antibody binding) can require the synthesis of thousands of different antibodies, which can be costly and time-consuming. Because the antibodies of the present invention are obtained from patients who have either been infected by a pathogen displaying the antigen or who have been vaccinated with an antigen, they have by their very nature and definition already undergone affinity maturation, as is most clearly demonstrated by the sequences of their CDR3 regions.

The present invention seeks to overcome the prior art disadvantages.

According to the present invention there is provided a method for identifying candidate sequences of at least the CDR3 region of antibodies specific against at least one antigen produced by a micro-organism during an infection or against a vaccine, comprising the steps of:
(i) with B cells isolated from at least one patient who has been infected by said micro-organism or administered said vaccine, sequencing at least the CDR3 region of the VH and/or VL coding regions of said B cells; and
(ii) correlating said sequenced at least the CDR3 regions of the VH and VL coding regions of said B cells to identify a set of candidate sequences for at least a CDR3 region of antibodies specific against said at least one antigen produced by said micro-organism or against said vaccine, each of said set of candidate CDR3 sequences or a sequence having at least 60% homology therewith occurring in total at a frequency of at least 1 percent in the set of sequences determined at step (i).

Examples of patients used as a source of B cells in such a method are humans and other mammals such as mice, rabbits, rats, baboons, monkeys and apes.

In certain embodiments of the invention, step (i) may comprise the steps of:
(i)(a) isolating B cells from at least one patient who has been infected by said micro-organism or administered said vaccine; and
(i)(b) sequencing at least the CDR3 region of the VH and/or VL coding regions of said B cells;

The sequences occurring in total at a frequency of at least 1 percent in the set of sequences determined at step (i) may be the candidate CDR3 sequences or a sequence having at least 80% homology, for example 85, 90, 95, 96, 97, 98 or 99% homology therewith. Sequence homology is as determined using the BLAST2 program (Tatusova TA *et al.,* FEMS Microbiol Lett. 1999 May 15;174(2):247-50; PMID: 10339815) at the National Center for Biotechnology Information, USA (www.ncbi.nlm.nih.gov) with default parameters.

For example, the sequences occurring in total at a frequency of at least 1 percent in the set of sequences determined at step (i) may be the candidate CDR3 sequences or a sequence having 1 or 2 amino acid changes therefrom. For example, a first sequence may occur at a frequency of 0.7 percent, and first, second, third and fourth sequences each having a single amino acid change therefrom each occur at a frequency of 0.1% - the total occurrence is therefore 1.1% and the dominant antibody sequence (occurring at a frequency of 0.5%) is therefore a candidate CDR3 sequence.

This is fundamentally different to prior art methods of making a library of antibodies in a vector such as a phage library where antibodies are isolated by panning and which require binding with a specific (known) antigen in a structurally- and charge-specific state. An analogy by which the two can be compared would be to say that the prior art making of a library and subsequent panning is like going into a library, picking up a book and hoping that it is the right one. In comparison, the present invention is like going into a library, reading all of the books in the library and finding that one book is dominant - that there are many copies of it - and that it is relevant to the disease to be treated.

In addition, the prior art use of library systems has a number of significant problems - in particular, some antibodies produced by a library may cause the death of the organism expressing them and therefore they simply cannot be detected. This is not a particular problem when e.g. looking for antibodies specific to cancers, but when one is searching for antibodies specific to an antigen from a pathogen which might be homologous to one produced by the host expression system (e.g. *E. coli)* then important antibodies cannot be expressed. The use of e.g. *E. coli* to express libraries of e.g. human antibodies also suffers from the problem of codon usage - codons used by humans for specific amino acids can frequently not be the optimum ones for the same amino acid in *E. coli* or other host systems. This means that an important antibody might not be expressed (or at least not in sufficient quantities) since the codons in its sequence are highly inefficient in *E. coli,* resulting in the *E*. *coli* being unable to read through and express it in full. Codon optimisation of antibody libraries is obviously not an option since the libraries would first have to be sequenced, which defeats the main advantages of using libraries. Since the present invention sequences antibodies directly from a patient, it avoids this problem.

The correlation step (ii) may also correlate the occurrence of the candidate sequences against their occurrence in patients who have not been infected with the micro-organism or administered the vaccine, sequences only being determined to be candidate sequences if they or a sequence having one or two amino acid changes from them occur in total at a frequency of less than 1 percent in the non-infected/vaccinated patients.

The at least one antigen produced by the micro-organism may of course be an immunogen.

The present invention provides unique opportunities for understanding antibody responses to infection which were not previously available, and provides novel diagnostic and therapeutic opportunities.

In particular, the methods of the present invention bypass the need to isolate an antigen from the micro-organism, and instead determine the identity of antibodies specific against one or more antigens produced by the micro-organism As is explained below, the methods of the present invention allow the identification of therapeutically effective antibodies and can allow the identification of the most important parts of antibody sequences. This is particularly evident when the B cells of a given patient are sampled at different time points during the course of an infection by a micro-organism - as the patient's immune system becomes focussed on producing therapeutically effective antibodies against the infecting micro-organism, a focussing of antibody sequences is observed, and the development of variable and non-variable regions within the CDR parts of the VH and VL sequences is observed. In addition, the methods of the present invention can allow the identification of antibodies best suited to the treatment of infection in particular groups of patients, such as age, sex and racial groups. Similarly by comparing the antibody sequences of patients who have recovered from infection with those that have not recovered from infection, ineffective antibody sequences (which might be produced by both sets of patients) can be identified.

The B cells isolated from the at least one patient can be peripheral B-cell lymphocytes (PBLs), and can be isolated from a blood sample from the at least one patient. B-cells can also be isolated from other sources and the present invention extends to their use. For example, B-cells can be isolated from spleen.

Antibody CDR sequences are determined as detailed in the "Experiments" section using standard techniques and definitions of their start and end.

As is detailed below, the antibody sequences are determined from patients with specified infections. These sequences can be derived from B cell immunoglobulin mRNA and hence reflect expressed antibodies and repertoires therein. The sequences determined according to the present invention need not be the sequences of whole antibody molecules - they comprise at least the VH and VL sequences which specify the regions responsible for antigen binding.

From the nucleotide sequences determined by the initial sequencing, putative amino acid sequences for the VH and VL regions can be determined using standard algorithms and software packages (e.g. see www.mrc-Imb.cam.ac.uk/pubseq/, the Staden package and Gap4 programs). These can be further characterised to determine the CDR (Complementarity Determining Region) parts of the VH and VL sequences, particularly CDR1, CDR2 and CDR3. Methods for determining the putative amino acid sequences and identifying CDR regions are well known and detailed below.

As well as the VH and/or VL amino acid sequences, several pieces of additional information can be used in the correlation step:
(i) the micro-organism causing the patient's infection;
(ii) the site of infection;
(iii) the time point during the infection process at which antibody sequences were sampled
(iv) patient details - none or more of: sex, race, and age; and
(v) the range of complementarity determining regions (CDR) ie the variable regions of the antibody that undergo direct antigen binding/contact.

Thus in a method according to the present invention, B cells can be used which have been isolated from the at least one patient at a plurality of time points during infection of the at least one patient by the micro-organism (or post-vaccination), correlation step (ii) correlating the time point during infection of the at least one patient by the micro-organism at which the B cells are isolated.

Similarly, in a method according to the present invention, B cells can be used which have been isolated from at least two patients, at least one of whom has recovered from infection by the micro-organism, and at least one of whom has not recovered from infection by the micro-organism, correlation step (ii) correlating the recovery of the at least two patients from infection by the micro-organism.

Similarly, in a method according to the present invention, B cells can be used which have been isolated from at least two patients, the patients being infected by different micro-organisms producing the at least one antigen, correlation step (ii) correlating the sequenced at least the VH and VL coding regions of the B cells to identify a set of candidate sequences for antibodies, each of which is specific against at least one shared antigen produced by the different micro-organisms or is specific against different antigens produced by the different micro-organisms.

The sequencing of the VH and VL regions can also be used to identify the surrounding antibody framework, and this can be used to determine the antibody isotype. This can then be used in the correlation step to determine whether specific antibody isotypes are particularly useful.

Experiments have been performed to sequence the VH and VL regions of PBLs isolated from patients with methicillin-resistant *Staphylococcus aureus* (MRSA), *Candida albicans, Pseudomonas aeruginosa* (the causative agent of cystic fibrosis), *Streptococcus oralis* and vancomycin-resistant Enterococci (VRE) infections and to identify antibody sequences which are associated with resistance to the infections and which can therefore be used to effect therapy against those infections. These sequences have formed the basis of GB 0127983 and WO 01/76627.

For all of these, the isolated VH or VL sequences demonstrate skewing of the antibody repertoire over the course of infection, thus revealing the identity of matured, immunity-conferring VH and VL sequences. This has typically been done using 5,000 to 15,000 sequences for each VH or VL. VH and VL repertoires from healthy individuals can also be used for comparison purposes.

By the term "therapy" is meant any treatment which is designed to cure, alleviate, remove or lessen the symptoms of, or prevent or reduce the possibility of contracting any disorder or malfunction of the human or animal body

The invention is suitable for identifying antibody sequence useful in treating the above infections, as well as any other infections such as viral infections where antibody has been shown to be important (including but not limited to HIV, influenza virus, hepatitis viruses A, B, C, and D, haemorrhagic viruses such as Ebola, as well as Dengue and Yellow Fever viruses) as well as parasitic infections such as malaria *(P. falciparum).*

Using the abovementioned additional information fields in the correlation step (ii), it is easy to perform the correlation using e.g. only CDR sequences, CDRs from either a given patient or a given infection or both, CDRs from a given site or patient group, or CDRs from different sampling times over the course of infection.

These correlations are CDR-specific because these are the hypervariable regions responsible for antigen binding and sequence diversity of the antibody repertoire (although the framework regions can be used in the correlation step as well to reveal the antibody isotype).

The present invention is useful in a wide range of applications, e.g. antibody therapy, and vaccine studies.

### Antibody Therapy:

This involves the passive transfer of antibody to non-immune individuals (e.g. patients undergoing chemo/radio therapy, immunosuppression for organ transplantation, immunocompromised due to underlying conditions such as diabetes, trauma etc, also the very young or very old).

The present invention can be used to determine the sequences of antibodies conferring immunity by looking for over-represented VH and VL sequences in patients who have overcome infection. These protective antibodies can be re-synthesised at the genetic level, over-expressed in *E. coli* (or other expression systems) and purified. The resultant purified recombinant antibody can then be administered to patients as a passive immunotherapy. Antibodies can also be ordered from commercial suppliers such as Operon Technologies Inc., USA (www.operon.com) by simply supplying them with the sequence of the antibody to be manufactured.

Therapeutically useful sequences can be identified in a number of ways which can be used in the correlation step (ii):
1) By looking for antibody sequence over-representation in patients who have recovered from infection - B cells that produce antibodies capable of pathogen binding undergo clonal expansion, hence high frequency antibody sequences are most likely to bind pathogen and confer immunity.
2) By following alterations in the antibody repertoire over the course of infection. During infection, antibodies undergo a maturation process to improve pathogen binding and this is characterised by sequence alterations. Also, B cell clonal expansion is more prevalent in the final stages of infection where the infection is cleared. The most frequent antibodies in the repertoire are chosen as candidates for immunotherapy. Following the maturation process the candidate antibody will demonstrate which key amino acid residue alterations improve antigen binding and this information can be used to improve antibody design.
3) Analysis of the repertoires from different patients with the same infection can identify shared and identical protective antibodies. These antibodies are attractive choices for immunotherapy as their occurrence in different patients suggests a strong positive selection in their favour, hence an important role in antibody-based immunity.
4) Analysis of the repertoires from patients with different infections. In this situation, if a common antibody sequence is found in the repertoires for both pathogens, the antibody may be useful in treating both types of infections, ie displaying a broad spectrum.
5) Analysis of the repertoires for affinity maturation of sequences.

### Vaccination Studies:

Vaccination protects against infection by priming the immune system with pathogen-derived antigen(s). Vaccination is effected by a single or repeated exposures to the pathogen-derived antigen(s) and allows antibody maturation and B cell clonal expansion without the deleterious effects of the full-blown infectious process. T cell involvement is also of great importance in effecting vaccination of patients. The present invention can be used to monitor the immunisation process with experimental vaccines. Subjects are given the experimental vaccine and VH and VL sequences are amplified from the patient and the antibody repertoire analysed as described above. Qualitative and quantitative assessment of the vaccination process is possible:
1) The VH/VL repertoire of a group of patients who have been administered an experimental vaccine is assessed. A precise molecular dissection of the resulting antibody response to the vaccine can be performed to determine (a) clonal expansion of protective antibodies, (b) protective antibody production in different populations (for example differing ethnic groups with different genetic backgrounds, as well as e.g. age and sex groups), and (c) the long term effect of the vaccine i.e. the antibody response over the long term - long term antibody memory in the immune system as well as any autoimmune defects;
2) Where vaccination results in an increased frequency of a given antibody, the antibody sequence can easily be cloned and expressed and used in animal models of infection. If the antibody is protective, it may be useful in itself as an immunotherapy, but this also demonstrates that the vaccine is likely to be protective without subjecting the human subject to experimental infection. Alternatively, in the absence of a suitable animal model, the cloned protective antibody can be assessed in cell cultures (particularly useful for human studies with HIV and other viruses). This is a unique approach for studying vaccine efficacy.

Also provided to the present invention is a method of manufacture of a medicament for the treatment of an infection by a micro-organism which produces at least one antigen, comprising the steps of:
(i) performing a method according to the present invention to identify a set of candidate sequences for antibodies specific against the at least one antigen produced by the micro-organism; and
(ii) synthesising at least one antibody comprising a candidate sequence specific against the at least one antigen produced by the micro-organism.

Such a medicament may of course incorporate a pharmaceutically acceptable carrier, diluent or excipient (Remington's Pharmaceutical Sciences and US Pharmacopoeia, 1984, Mack Publishing Company, Easton, PA, USA; United States Pharmacopoeia, ISBN: 1889788031).

Also provided according to the present invention is a method of treatment of an infection of a patient by a micro-organism which produces at least one antigen, comprising the steps of:
(i) performing a method according to the present invention to identify a set of candidate sequences for antibodies specific against the at least one antigen produced by the micro-organism;
(ii) synthesising at least one antibody comprising a candidate sequence specific against the at least one antigen produced by the micro-organism; and
(iii) administering a therapeutically effective quantity of the at least one synthesised antibody to the patient

Also provided according to the present invention is a method of producing a database which identifies candidate sequences for antibodies specific against at least one antigen produced by a micro-organism, comprising the steps of:
(i) performing a method according to the present invention to identify a set of candidate sequences for antibodies specific against the at least one antigen produced by the micro-organism; and
(ii) storing the data produced by said method in the database.

Also provided is a method of generating a report which identifies candidate sequences for antibodies specific against at least one antigen produced by a micro-organism, comprising the steps of:
(i) performing a method according to the present invention to identify a set of candidate sequences for antibodies specific against the at least one antigen produced by the micro-organism; and
(ii) producing a report comprising the data produced by the method of step (i).

Also provided according to the present invention is a method for determining the efficacy of a vaccine, comprising the steps of:
(i) with B cells isolated from at least one patient who has been administering said vaccine, sequencing at least the CDR3 region of the VH and/or VL coding regions of said B cells; and
(ii) correlating said sequenced at least the CDR3 region of the VH and/or VL coding regions of said B cells to identify a set of candidate sequences for at least the CDR3 region of antibodies specific against said vaccine, each of said set of CDR3 candidate sequences or a sequence having at least 60% homology therewith occurring in total at a frequency of at least 1 percent in the set of sequences determined at step (i).

In certain embodiments of the invention, step (i) above may comprise the steps of:
(i)(a) administering said vaccine to at least one patient;
(i)(b) isolating B cells from said at least one patient; and
(i)(c) sequencing at least the CDR3 region of the VH and/or VL coding regions of said B cells.

Correlation step (ii) may comprise correlating said sequenced at least a CDR3 region of the VH and VL coding regions of said B cells with sequenced at least a CDR3 region of the VH and VL coding regions of B cells isolated from at least one patient who has been infected with a micro-organism against which vaccination with said vaccine is intended to stimulate a protective immune response.

As described above, additional information may be used in the correlation of step (ii), including:
(i) the time since administration of the vaccine to the patient;
(ii) patient details - none or more of: sex, race, and age; and
(iii) the range of complementarity determining regions (CDR) ie the variable regions of the antibody that undergo direct antigen binding/contact.

The antibody sequences determined from patients who have been administered the vaccine can be compared with antibody sequences isolated from patients who have been infected with the micro-organism against which the vaccine is intended to stimulate a protective immune response in patients. Thus it is possible to determine whether the vaccine results in the generation of antibodies which are also produced in response to infection by the micro-organism itself. In particular, the comparison can be made using antibody sequences determined from patients who have recovered from infection by the micro-organism.

In particular the method may determine the efficacy of the vaccine in stimulating a protective immune response against the micro-organism against which vaccination with the vaccine is intended to stimulate a protective immune response.

Also provided according to the present invention is a method of vaccinating a patient against infection by a micro-organism, comprising the steps of:
(i) performing with at least one vaccine a method according to the present invention to determine the efficacy of the at least one vaccine in stimulating a protective immune response against the micro-organism against which vaccination with the at least one vaccine is intended to stimulate a protective immune response;
(ii) correlating the results of step (i) to determine the most suitable of the at least one vaccine for the patient; and
(iii) vaccinating the patient with the most suitable of the at least one vaccine.

Also provided is a method of producing a database which identifies the efficacy of a vaccine, comprising the steps of:
(i) performing a method according to the present invention to determine the efficacy of the vaccine; and
(ii) storing the data produced by the method in the database.

Also provided according to the present invention is a method of generating a report which identifies the efficacy of a vaccine, comprising the steps of:
(i) performing a method according to the present invention to determine the efficacy of the vaccine; and
(ii) producing a report comprising the data produced by the method.

As regards antibodies usable in the present invention, particularly synthetic antibodies (the term "antibody" is also considered to incorporate antigen binding fragments of antibodies unless the context otherwise requires), the antibody may be a whole antibody or an antigen binding fragment thereof and may in general belong to any immunoglobulin class. Thus, for example, it may be an IgM or an IgG antibody. The antibody or fragment may be of animal, for example, mammalian origin and may be for example of murine, rat, sheep or human origin. It may be a natural antibody or a fragment thereof, or, if desired, a recombinant antibody fragment, i.e. an antibody or antibody fragment which has been produced using recombinant DNA techniques.

Particular recombinant antibodies or antibody fragments include, (1) those having an antigen binding site at least part of which is derived from a different antibody, for example those in which the hypervariable or complementarity determining regions of one antibody have been grafted into the variable framework regions of a second, different antibody (as described in, for example, EP 239400); (2) recombinant antibodies or fragments wherein non-Fv sequences have been substituted by non-Fv sequences from other, different antibodies (as described in, for example, EP 171496, EP 173494 and EP 194276); or (3) recombinant antibodies or fragments possessing substantially the structure of a natural immunoglobulin but wherein the hinge region has a different number of cysteine residues from that found in the natural immunoglobulin but wherein one or more cysteine residues in a surface pocket of the recombinant antibody or fragment is in the place of another amino acid residue present in the natural immunoglobulin (as described in, for example, WO 89/01782 and WO 89/01974).

Teachings of texts such as Harlow, E. and Lane, D. ("Using Antibodies: A Laboratory Manual", Cold Spring Harbor Laboratory Press, New York, 1998) further details antibodies, antibody fragments, their preparation and use.

The antibody or antibody fragment may be of polyclonal or monoclonal origin. It may be specific for at least one epitope.

Antigen binding antibody fragments include, for example, fragments derived by proteolytic cleavage of a whole antibody, such as F(ab')2, Fab' or Fab fragments, or fragments obtained by recombinant DNA techniques, for example Fv fragments (as described, for example, in WO 89/02465).

The antibodies according to the invention may be prepared using well-known immunological techniques. Thus, for example, any suitable host may be injected with the protein and the blood collected to yield the desired polyclonal antibody after appropriate purification and/or concentration (for example by affinity chromatography using the immobilised protein as the affinity medium). Alternatively splenocytes or lymphocytes may be recovered from the protein-injected host and immortalised using for example the method of Kohler *et al.* (1976, Eur. J. Immunol., 6: 511), the resulting cells being segregated to obtain a single genetic line producing monoclonal antibodies. Antibody fragments may be produced using conventional techniques, for example, by enzymatic digestion with pepsin or papain. Where it is desired to produce recombinant antibodies according to the invention these may be produced using, for example, the methods described in EP 171469, EP 173494, EP 194276 and EP 239400.

Antibodies according to the invention may be labelled with a detectable label or may be conjugated with an effector molecule, for example a drug eg. an antibacterial agent or a toxin or an enzyme, using conventional procedures and the invention extends to such labelled antibodies or antibody conjugates.

The contents of each of the references discussed herein, including the references cited therein, are herein incorporated by reference in their entirety.

Where "PMID:" reference numbers are given for publications, these are the PubMed identification numbers allocated to them by the US National Library of Medicine, from which full bibliographic information and abstract for each publication is available at www.ncbi.nlm.nih.gov.

The invention will be further apparent from the following description, with reference to the several figures of the accompanying drawings, which show, by way of example only, forms of identifying candidate sequences for antibodies specific against at least one antigen and of determining the efficacy of a vaccine.

### Of the Figures:

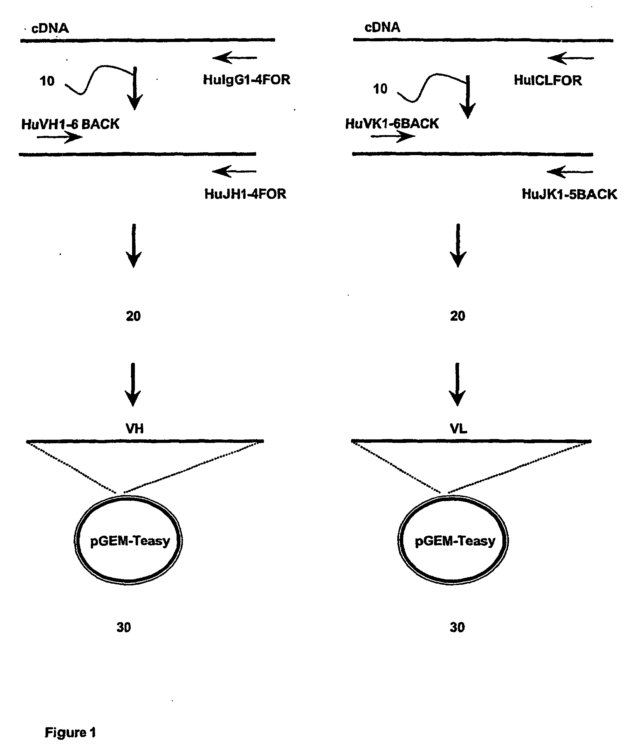

- Figure 1: shows the general principles for the isolation and DNA sequencing of VH and VL antibody gene fragments from B cells. Reference numeral 10 indicates primary PCR, reference numeral 20 the cloning into a DNA sequencing vector using the T-tailing principle - random orientation, and reference number 30 the determining of the nucleotide sequence of forward and reverse strands using M13 forward and reverse primers; and

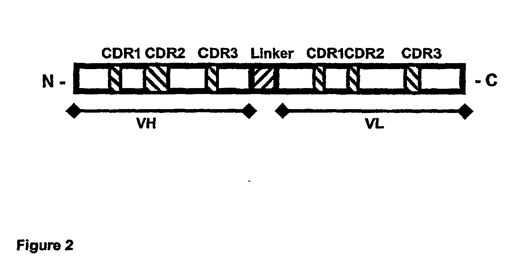

- Figure 2: shows a schematic depiction of resynthesised recombinant antibody gene cassette. VH and VL regions are linked with a glycine serine-rich linker. Each variable domain contains three Complementarity Determining Regions (CDRs) which participate in antigen binding.

### EXPERIMENTS

The experiments below detail methods for identifying antibody VH and VL sequences that confer immunity in patients with viral, bacterial or parasitic infection. These sequences are then used to produce synthetic antibodies and these synthetic antibodies are suitable for administration to patients for therapy.

Also detailed are methods of determining the efficacy of a vaccine in generating a desired immune response in a patient

Unless stated otherwise, all procedures were performed using standard protocols and following manufacturer's instructions where applicable. Standard protocols for various techniques including PCR, molecular cloning, manipulation and sequencing, the manufacture of antibodies, epitope mapping and mimotope design, cell culturing and phage display, are described in texts such as McPherson, M.J. *et al.* (1991, PCR: A practical approach, Oxford University Press, Oxford), Sambrook, J. *et al.* (1989, Molecular cloning: a laboratory manual, Cold Spring Harbour Laboratory, New York), Huynh and Davies (1985, "DNA Cloning Vol I - A Practical Approach", IRL Press, Oxford, Ed. D.M. Glover), Sanger, F. *et al.* (1977, PNAS USA 74(12): 5463-5467), Harlow, E. and Lane, D. ("Using Antibodies: A Laboratory Manual", Cold Spring Harbor Laboratory Press, New York, 1998), Jung, G. and Beck-Sickinger, A.G. (1992, Angew. Chem. Int. Ed. Eng., 31: 367-486), Harris, M.A. and Rae, I.F. ("General Techniques of Cell Culture", 1997, Cambridge University Press, ISBN 0521 573645), "Phage Display of Peptides and Proteins: A Laboratory Manual" (Eds. Kay, B.K., Winter, J., and McCafferty, J., Academic Press Inc., 1996, ISBN 0-12-402380-0).

Reagents and equipment useful in, amongst others, the methods detailed herein are available from the likes of Amersham (www.amersham.co.uk), Boehringer Mannheim (www.boehringer-ingeltheim.com), Clontech (www.clontech.com), Genosys (www.genosys.com), Millipore (www.millipore.com), Novagen (www.novagen.com), Perkin Elmer (www.perkinelmer.com), Pharmacia (www.pharmacia.com), Promega (www.promega.com), Qiagen (www.qiagen.com), Sigma (www.sigma-aldrich.com) and Stratagene (www.stratagene.com).

The term "antibody" in its various grammatical forms is used herein to refer to immunoglobulin molecules and immunologically active portions of immunoglobulin molecules, i.e., molecules that contain an antibody combining site or paratope. Such molecules are also referred to as "antigen binding fragments" of immunoglobulin molecules.

Illustrative antibody molecules are intact immunoglobulin molecules, substantially intact immunoglobulin molecules and those portions of an immunoglobulin molecule that contain the paratope, including those portions known in the art as Fab, Fab', F(ab')2, scFv and F(v).

The term "antibody combining site" refers to that structural portion of an antibody molecule comprised of a heavy and light chain variable and hypervariable regions that specifically binds (immunoreacts with) antigen.

The identification and sequencing ofB cells producing antibodies and the analysis of the resulting data comprises the following basic steps:
1) Isolation of VH and VL coding regions from circulatory B cells of human patients.
2) Determining the Nucleotide sequence of VH and VL repertoires.
3) Determination of VH and VL primary amino acid sequences.
4) Extraction of CDR *regions in silico -* incorporation into the database
5) Detection of dominant CDR & framework regions in VH or VL repertoire.
6) Construction & Production of therapeutic recombinant antibodies from dominant antibody sequences.

### 1. Isolation of VH and VL coding regions from circulatory B cells of human patients

For this method, human patients with a given microbial infection(s) were selected as donors of immunised B cells. The criteria for selection were:
(a) The patient must exhibit a pronounced antibody response to the pathogen in question, detectable for example by Western blotting. Samples of antibodies were collected from a patient blood sample. Samples were diluted and tested for immunoreactivity by Western blotting using antigen(s) derived from the microbial pathogen. In such an assay, patients exhibiting strong antibody responses showed pronounced recognition/antibody binding of microbial antigens as detected using anti-human polyclonal detection antibodies.
(b) The patient has survived the course of infection - this improves the chance of generating B cell responses for antibodies capable of neutralizing the pathogen.

Peripheral B-cell lymphocytes (PBLs) were collected from infected patient blood samples. For this heparinised blood was diluted in PBS (20 ml total) and overlaid onto a 15 ml cushion of Ficol Hypaque (Pharmacia; unless stated otherwise, all chemicals and culture media were purchased from Sigma, UK) in a 30 ml centrifuge tube. The PBLs were then collected by centrifugation (400 x g, 5 minutes) and washed in PBS and harvested by centrifugation again. RNA was prepared from PBLs using the QuickPrep mRNA purification kit (Pharmacia) exactly according to the manufacturers instructions. The isolated mRNA was used to prepare cDNA via a reverse transcriptase reaction (Promega cDNA synthesis kit). For this 2µg of mRNA was re-suspended in 16 µL nuclease-free water and heated to 65 °C for 3 minutes (to denature secondary structure) and then immediately chilled on ice for 1 minute. The mRNA was then added to the following cocktail: 8 µL 25 mM MgCl₂, 4 µL dNTP mix (10 mM with respect to each ribonucleotide triphosphate), 1 µL RNAsin 40 u µL⁻¹ stock solution, 1.2 µL AMV reverse transcriptase (25 u µL⁻¹ stock solution), 6 µL cDNA 10 pmol µL¹ primer (see Figure 1 - cDNA synthesis). The mixture was incubated at 42 °C for 1 hour and then incubated at 100 °C for 3 minutes to stop the reaction.

DNA coding for antigen binding regions was then amplified by PCR using the cDNA prepared from patients PBLs. For this, the cDNA was used in the following PCR reaction to produce either heavy chain or light chain-derived antibody variable region DNA (see Figure 1): 2.5 µL cDNA, 33 µL water, 4 µL dNTP mix (25 mM with respect to each deoxynucleotide triphosphate), 5 µL Taq reaction buffer (Perkin Elmer), 2.5 µL of an equimolar primer mix (final concentration of 20 pmol with respect to each primer) and 0.5 µL Taq DNA polymerase ( 1 u µL ⁻¹, Perkin Elmer Corp). The forward and reverse primers used in these reactions are depicted in Figure 1, and their respective nucleotide sequences are listed in Table 1. PCR reaction conditions were 94 °C for 1 minute, 57 °C for 1 minute and 72 °C for 2 minutes for a total of 30 cycles, with an extended denaturation (94°C for 5 minutes) prior to cycle 1 and an additional extension step after the end of cycle 30 (72 °C for 10 minutes). PCR was performed using a Perkin Elmer 9700 GeneAmp PCR machine. 5 µL of the PCR reaction was run on a 1% agarose gel to check the amplification of the expected 393 base pair (bp) product The remaining product was run on a 0.8% low melting point (LMP) agarose gel and the 393 bp band was excised using a clean scalpel blade. DNA was extracted from the agarose gel slice using a GeneClean II Kit (Anachem, Luton, UK).

The PCR resulted in two fragment types -derived from VH and VL regions respectively, depending on the PCR primer sets used (for details of primers sets for antibody genes and a PCR schematic see Table 1 and Figure 1, respectively).

VH and VL gene fragments were cloned into cloning vector pGEM-T easy (Promega Corporation) to facilitate DNA sequencing. For this 3 µg PCR product was prepared for restriction using QIAquick PCR purification spin columns (Qiagen, UK) according to the manufacturers instructions. DNA was eluted from the spin column in 40 µL buffer EB. Purified PCR product (2 µL) was mixed with 1 µL pGEM-T easy vector, 6 µL water and 1 µL DNA ligase and the mixture ligated for 1 h at room temperature. Ligations were then transformed into electrocompetent *E. coli* TG1 cells (Stratagene) by electroporation, and plated out onto agar plates containing Ampicillin 100 µg ml⁻¹ IPTG (100 µM) and X-gal (0.006 % w/v). Colonies were allowed to grow overnight at 37 °C and then stored at 4 °C. Recombinant colonies are identified as white colonies on this media.

### 2. Determining the nucleotide sequence of VH and VL repertoires

DNA was first prepared from VH and VL recombinant *E. coli* clones. For this, individual colonies were each transferred to 1.2 ml LB broth supplemented with Ampicillin 100 µg ml⁻¹ using a 96 well plate format. Cultures were the grown at 37°C for 24 hours. Bacterial cells were harvested by centrifugation at 4,000 x g, 30 minutes and the supernatants discarded. Plasmid DNA was prepared using Wizard SV 96 plasmid purification kits (Promega Corporation) essentially following the manufacturer's instructions. Yields of plasmid DNA were typically in the order of 5 µg per 1.2 ml starter culture.

DNA sequencing reactions were performed using the DYEnamic ET dye terminator cycle sequencing kit (Amersham Pharmacia Biotech). Purified plasmid DNA (0.5 µg) was mixed with 8 µL DYEnamic ET terminator reagent premix and 1 µL M13 forward or reverse primer (5 µM) in a total reaction volume of 20 µL. Thermal cycling was then performed using a GeneAmp PCR system 9700 (Perkin Elmer) with the following parameters: 95 °C, 20 seconds; 50 °C, 15 seconds; 60 °C, 1 minute; 30 cycles). Reactions were performed using 96 well format non-skirted ELISA plates (AB Gene). Unincorporated dye terminator were removed using precipitation. For this, ethanol samples were mixed with 2 µL 7.5 M ammonium acetate and 55 µL of 100 % ethanol and centrifuged at 3000 g for 30 minutes. DNA pellets were washed with 70 % ethanol and re-suspended in 20 µL loading solution. Reactions were sequenced using a MegaBACE 1000 DNA sequencer (Amersham Pharmacia Biotech) following the manufacturers instructions (2 kV injection voltage for 30 s with electrophoresis at 6 kV for 200 minutes). Chromatograms are exported using the .scf file format for finishing and archiving.

### 3. Determination of VH and VL primary amino acid sequences

DNA sequences of VH and VL were determined in both forward and reverse strands (using M13 forward and reverse primers respectively) and compared in order to highlight discrepancies and maintain a high degree of accuracy. This was done using the Staden suite of programs (Staden, R. (1996) *The Staden Sequence Analysis Package.* Molecular Biotechnology 5, 233-241). First, sequence .scf files were entered into the PREGAP program where vector sequence was stripped off and the quality of the sequence was assessed. Poor quality sequences where the DNA sequence was ambiguous were rejected and re-sequenced. Forward and reverse strands were matched using the GAP program to highlight and resolve areas containing sequencing artefacts. The orientation of the VH or VL sequence was noted and reverse complemented where necessary to produce only forward reading frame orientations for translation. VH and VL gene sequences were then translated into amino acid sequences. Each sequence was given a unique identifier (name).

### 4. Extraction of CDR regions in silico - incorporation into the database

A general teaching of identifying CDR regions is at www.bioinf.org.uk/abs/

The following set of rules will allow the definition of the CDRs in an antibody sequence. There are rare examples where these virtually constant features do not occur (for example the human heavy chain sequence EU does not have Trp-Gly after CDR-H3). The Cys residues are the best-conserved feature.
**CDR-L1**
   - Start: Approx. residue 24
   - Residue before: always a Cys
   - Residue after: always a Trp. Typically Trp-Tyr-Gln, but also, Trp-Leu-Gln, Trp-Phe-Gln, Trp-Tyr-Leu
   - Length: 10 to 17 residues
**CDR-L2**
   - Start: always 16 residues after the end of CDR-L1
   - Residues before: generally Ile-Tyr, but also, Val-Tyr, Ile-Lys, Ile-Phe
   - Length: always 7 residues (except NEW (7FAB) which has a deletion in this region)
**CDR-L3**
   - Start: always 33 residues after end of CDR-L2 (except NEW (7FAB) which has the deletion at the end of CDR-L2)
   - Residue before: always Cys
   - Residues after: always Phe-Gly-XXX-Gly (SEQ ID NO: 69)
   - Length: 7 to 11 residues
**CDR-H1**
   - Start: Approx. residue 26 (always 4 after a Cys)
   - Residues before: always Cys-XXX-XXX-XXX (SEQ ID NO: 70)
   - Residues after: always a Trp. Typically Trp-Val, but also, Trp-Ile, Trp-Ala
   - Length: 10 to 12 residues
**CDR-H2**
   - Start: always 15 residues after the end of CDR-H1
   - Residues before: typically Leu-Glu-Trp-Ile-Gly (SEQ ID NO: 71), but a number of variations
   - Residues after: Lys/Arg-Leu/IIe/Val/Phe/Thr/Ala-Thr/Ser/IIe/Ala
   - Length: 16 to 19 residues;
**CDR-H3**
   - Start: always 33 residues after end of CDR-H2 (always 2 after a Cys)
   - Residues before: always Cys-XXX-XXX (typically Cys-Ala-Arg)
   - Residues after: always Trp-Gly-XXX-Gly (SEQ ID NO: 72)
   - Length: 3 to 25 residues

### 5. Detection of dominant CDR & framework regions in VH or VL repertoire

The database was constructed using SQL Server Database software (Microsoft). This allowed the database to be queried using SQL and allowed CDR1, CDR2 and CDR3 sequences to be extracted from any range of database VH or VL sequences in FASTA format Extracted CDRs were then subject to multiple alignment using CLUSTAL X in such a way so that identical or very similar CDRs are grouped together in blocks (Thompson, J.D., Higgins, D.G. and Gibson, T.J. (1994) CLUSTAL W: improving the sensitivity of progressive multiple sequence alignment through sequence weighting, positions-specific gap penalties and weight matrix choice. Nucleic Acids Research, 22:4673-4680). From this the frequency of recurring CDRs was determined and CDR-dominance in the immunoglobulin repertoire was established. Alternatively, a graphical interface was employed using Probiosys (proBionic EMEA, Amsterdam). ProBiosys prepares dendrograms from CLUSTAL .phy files and allows a rapid visual interpretation of the relationships among large numbers of aligned sequences. The whole process was performed for CDRs from the same patient sampled at different points during an illness, for CDRs from patients with different infections, or from sex-matched, age-matched or race-matched patient groups, depending on the search and the additional information added to the database entries.

### 6. Construction & Production of therapeutic recombinant antibodies from dominant antibody sequences

Once dominant antibody sequences were recognised in a given repertoire, the information was used to infer the presence of CDRs and frameworks that confer immunity. Selected VH and VL sequences were identified and their gene sequences resynthesised using synthetic oligonucleotides. This was important as it also allowed the human-derived gene sequence to be codon-optimised for *E. coli* in order to improve protein expression. Dominant VH and VL sequences were spliced together using a spacer (linker) sequence (Figure 2). This gene cassette, termed a scFv, was resynthesised to include terminal *Nde*I and *Not*I restriction sites for cloning into expression vector pET29b (Novagen). ScFv DNA was cut with *Nde*I (cuts in VH) or *Not* I (cuts in VL) using the following reaction: 40 µL DNA (4 µg), 10 µL of Restriction enzyme buffer D (Promega), 47 µL water and 2 µL *Nde*I and *Not*I enzyme (10 u µL⁻¹; Promega) with digestion for 4 h at 37 °C. DNA was then fractionated on 0.7% agarose TAE gels and the digested DNA excised from the gel and purified from the agarose slice using the Geneclean II kit (Bio 101) exactly according to the manufacturers instruction. pET29b vector DNA was cut with *Nde*I and *Not*I as described above. 1 µg vector was mixed with 1 µg restricted VL DNA resuspended in 8.5 µL water and ligated by addition of 1 µL 10 x ligation buffer (Boehringer Mannheim) and 0.5 µL DNA ligase (3 u µL⁻¹; Boehringer Mannheim), followed by ligation overnight at 14 °C.

The entire ligation was transformed into *E. coli* TG2 cells (Stratagene, UK) by electroporation. Transformants were then plated out onto agar plates containing Tetracycline (25 µg ml⁻¹ - selective for *recA* ::Tn10 on the chromosome of TG2) and Kanamycin (50 µg ml⁻¹ - selective for *pET29).* Recombinant plasmid DNA prepared from individual clones was checked for scFv sequence by digestion with *Nde*I and *Not*I as described above.

Recombinant scFvs were over-expressed in *E. coli* strain JM109(DE3) and purified thus enabling biochemical and biological characterisation. Recombinants were spread on LB plates with 50 mg/ml kanamycin and a single colony used to inoculate a 10 microlitre starter culture of LB broth with 50 micrograms per ml kanamycin. For this, a 1 L expression culture was prepared in the presence of kanamycin for 4-5 h at 37 °C with shaking at 300 rpm. At OD₆₀₀ =1, induction was performed using IPTG and the cells were grown with vigorous aeration for a further 3-5 h. Cells were then harvested by centrifugation (4000 x g, 15 minutes, 4 °C). The cell pellet was resuspended in 10 ml fresh (4 °C) lysis buffer (below) and stored at -20°C overnight prior to cell breakage using the 25 ml X-Press system (AB Biox)

### LYSIS BUFFER

50 mM Tris HCl pH 8.0
1 mM EDTA
100 mM KCl
0.1 mM AEBSF (amino ethyl benzene sulphonic acid)

The lysate was centrifuged in an Oakridge tube (24 300 x g, 4 °C, 30 minutes). The pellet was resuspended in 20 ml of ice cold lysis buffer on ice using a Silverson lab blender. The lysate was split into 8 x Oakridge tubes and each aliquot was diluted to 30 ml (total 120 ml) in ice-cold Lysis Buffer. Inclusion bodies were pelleted (24 300 x *g*, 4 °C, 30 minutes) and the supernatant was discarded. Each of the 8 pellets was resuspended in 30 ml of lysis buffer, and the inclusion bodies were harvested by centrifugation again (24 300 x g, 4 °C, 30 minutes). This wash/centrifugation step was performed 5 x times in total to clean the inclusion body fraction. Each pellet was resuspended in 15 ml of ice-cold water and the inclusion bodies were stored at -20 °C.

For refolding, 200 ml solution of 2% (w/v) N-lauryl sarcosine (NLS) in 50 mM Tris HCl pH 9.0 was prepared as follows. 4 g NLS was solubilised in 100 ml water with stirring. 10 ml of 1M Tris pH 9.0 stock solution was added and made up to 195 ml with water. 5 ml of inclusion body slurry was added and stirred vigorously for 30 minutes at room temperature.

CuCl₂ was added to a concentration of 100 µM. This serves as a catalyst for oxidation. The refolding reaction was transferred to 4 °C and stirred vigorously for 2 days to promote aeration. The refolding reaction was vacuum filtered through a 0.44 µm vacucup bottle top filter unit (90 mm diameter, Gellman Sciences) and the filtrate transferred to a Pellicon Labscale TFF system fitted with PLGC10 membrane unit (Millipore). The reaction was concentrated to 25 ml using tangential flow, discarding the permeate (the scFv is localised to the retentate). The solution was diafiltered against 40 x turn-over volumes (1 L) of 10 mM ammonium acetate (AAT) pH 9.0. Finally, the volume of the antibody was diluted to 50 ml using 10 mM AAT pH 9.0. The buffer exchanged antibody was stored for 2 hours at 4 °C. The typical protein content was 1 - 2 mg ml⁻¹ with a yield of up to 50 mg per litre.

For scFv purification a 10 ml bed volume of Ni NTA superflow agarose Qiagen was prepared according to the manufacturers instructions using a 10 ml glass column (Sigma) without flow adaptors. The column was equilibrated with 50 ml Buffer B (6M urea, 0.1 M NaH₂PO₄, 10 mM Tris HCl pH 8.0). Refolded scFv (as described above) was diluted 1/5 in Buffer B and applied directly to the column at ml min⁻¹. 50 ml buffer B was applied to the column (flow rate 5 ml min⁻¹) followed by 50 ml Buffer C (same composition as buffer B but pH 6.3). The purified scFv was eluted from the column by slowly applying Buffer B supplemented with 250 mM high grade imidazole until the A₂₈₀ < 0.05.

### Results:

The variable heavy chain (VH) of the antibody genes from peripheral blood lymphocytes have been TA cloned and sequenced. In each case the third complementarity determining region (CDR3) was identified. This region is both the most variable region of the VH chain and the area identified as being most important in determining antigen binding, for this reason this area is used as the signature for each VH chain. Several libraries of sequence data have been developed. M1 is a library of VH sequences from a patient with septicaemia caused by a Methicillin Resistant *Staphylococcus aureus* (MRSA). M2 is a library from a different patient with long standing MRSA septicaemia, this sample was taken at the very end of the infectious period. M3 is another library constructed from the same patient as in M2 but taken ten days later than the M2 sample. P1 is a library from a Cystic Fibrosis (CF) patient colonized long term with *Pseudomonas aeruginosa.* C1 is a library from a patient infected with systemic *Candida albicans* infection.

Each library has a small number of dominant antibodies. The most common antibody in each library have a frequency of 10% (SEQ ID NO: 27) (RNNWPPT), 41% (SEQ ID NO: 28) (RSGSHYDAFNI), 32% (SEQ ID NO: 29) (VTGCFDH), 7% (SEQ ID NO: 30) (LHGFGHGFRI) and 30% (SEQ ID NO: 31) (GEIFDY) (M1, M2, M3, P1, C1 respectively). M1 has two major VH chains accounting for 18% of the library. M2 has four dominant VH chains that account for 80% of the library. M3 has four VH chains making-up 79% of the library. P1 has 3 VH chains accounting for 18% of the library. The C1 library has six VH chains which together account for 72% of the clones in the library. Various of these VH chains have been tested in mouse models and shown to have therapeutic activity against the relevant agent.

M2 and M3 library are comparisons of the VH repertoire over a period of time for an individual with an MRSA septicaemia. The most dominant VH chain (SEQ ID NO: 28) (RSGSHYDAFNI) is at a frequency of 41 % at the end of the infectious period (M2); this has dropped to 22% after 10 days (M3). Other common antibodies show similar drops 19% to 7% (SEQ ID NO: 32) (LLGGSSGRYMDV), 10% to 2% (SEQ ID NO: 33) (HRGGPQCHNLNCYTLDF) and 7% to 3% (SEQ ID NO: 34) (EAESYAERIGFDY). Other antibodies show a rise in levels over the same time period. The most dominant antibody in the M3 library (SEQ ID NO: 29) (VTGCFDH) with a frequency of 32% has so far not been detected in the M2 library. In this case VH chains that show a reduction in the post-infectious period would be considered potential therapeutic agents. Those antibodies that show a steep rise following clearing of the infection maybe of less importance as therapeutics. Analysis of the VH chain expression over the course of an infection and beyond is important in helping to identify neutralizing antibodies.

With the libraries so far sequenced it has also been possible to compare the VH chain repertoire of individuals with similar and different infections. M1 and M2/M3 are libraries from two different patients with the same infection. So far it has been possible to identify six different CDR3 sequences that are present in both MRSA patients. One such antibody (SEQ ID NO: 33) (LLGGSSGRYMDV) makes up 4% of the repertoire from the M1 library and 19% and 7% of the M2 and M3 libraries respectively. This antibody is identical along the whole length of the VH region in both patients. Four more different VH chains are also found to be present in both patients' libraries and have an identical sequence along the rest of the VH region (SEQ ID NOs: 35-38) (YDVVVTGKYAFDI, RRYHDLTTNNWFDP, NVGSGSYYTGGHWFDP, HRGGPQCHNLNCYTLDF). The fifth CDR3 shared by both patients (SEQ ID NO: 39) (ARRYGTSNYCFDH) is found at a frequency of 2% and 3% in libraries M1 and M2 respectively. The VH chains with this CDR3 are identical along most of the VH chain except for one important region, the second complementarity determining region (CDR2). The CDR2 is another region of the mature antibody that comes into contact with the antigen and plays an important role in its activity. Identification of antibodies with the same CDR3 but different sequences outside of this region will yield antibodies with similar target antigens although other properties may vary.

The libraries from patients with different infections have also been compared. These libraries have also been shown to share VH chains with identical CDR3 sequences although this appears to be less common. We have so far found one CDR3 that is found in both the P1 and M2 libraries (SEQ ID NO: 40) (DQSHAAQGF). Antibodies common to patients with different infections maybe a sign that the VH chain is not specific to the infection of interest In this case it is not possible to rule out that the P1 library patient has not come into contact with *S. aureus* as this is a common bacteria in the environment.

### Summary of results:

1) Individuals infected with bacteria show a very strong focusing of a small number of CDR sequences.
2) The levels expression of antibodies with particular CDR3 type varies over the course of infection and in the post infection period.
3) Patients with the same disease are shown to express antibodies with the same CDR3 sequences. In most cases these are the same for the whole length of the VH chain. In other cases variation is seen in other parts of the VH chain.
4) Patients with different infections can share antibodies with the same CDR3 and VH sequences.

### 7. Additional studies

Further studies were undertaken to identify CDR3 regions in patients who were VRE colonised, VRE blood culture positive, MRSA blood culture positive, and with a cystic fibrosis patient infected with *P. aeruginosa.*

Sequences of CDR 3 regions in the various libraries were determined as detailed above.

Results of the experiments are given in Table 2 (below) and show focussing of the VH repertoire of blood culture positive patients, a number of VH CDR3 sequences being very common. In Table 2, the SEQ column is the SEQ ID NO of the CDR 3 sequence. Other columns give results for the percentage of clone in each library. For V01 , n=75; V02, n=82; V03, n=110; V04, n=43; M01, n=103; M02, n=231; M03, n=150; M04, n=136; M05, n=229; P01, n=131.

### Library information

| | |
|---|---|
| V01 | VRE colonised patient |
| V02 | VRE blood culture positive |
| V03 | VRE blood culture positive |
| V04 | VRE blood culture positive |
| M01 | MRSA blood culture positive |
| M02 | MRSA blood culture positive |
| M03 | MRSA blood culture positive |
| M04 | MRSA blood culture positive |
| M05 | MRSA blood culture positive |
| P01 | *P.aeruginosa* CF patient |

M02, M03, M04 are three samples from the same patient over a time course

### General Points

1) All the blood culture positive patients show focussing of the VH repertoire. The most common VH CDR3 sequences from each of the blood culture positive libraries are:

| | | |
|---|---|---|
| V02 | (SEQ ID NO: 62) | 9% |
| V03 | (SEQ ID NO: 68) | 19% |
| V04 | (SEQ ID NO: 67) | 42% |
| M01 | (SEQ ID NO:40) | 13% |
| M02 | (SEQ ID NO: 45) | 38% |
| M03 | (SEQ ID NO:67) | 24% |
| M04 | (SEQ ID NO: 45) | 12% |
| M05 | (SEQ ID NO: 53) | 4% |

The non blood culture positive libraries, V01 and P01, remained relatively diverse. V01 has no notable focussing and the most common VH CDR3 from P01 (SEQ ID NO: 56) accounts for only 3% of the library.

2) On the whole libraries from patients with the same infection share more VH CDR3's than patients with different infections. The list of common antibodies shows that six different VH CDR3 sequences (SEQ ID NOs: 59,60,61,66,67 and 68) are shared by two different VRE blood culture positive patients. One of these six antibodies is found at a very low level (<1%) in P01 library and another at <1% in M04.

VH CDR3 sequences from the MRSA libraries show a similar result. One VH CDR3 sequence (SEQ ID NO: 46) is found in all three of the MRSA patients. Three other VH CDR3 sequences (SEQ ID NOs: 47, 48 and 50) appear in two different libraries. None of these VH CDR3 sequences appear in libraries from patients with other diseases.

### VRE Libraries

The VH from three VRE blood culture positive patients (libraries V02, V03 and V04), and from on patient colonised with VRE (V01) were sequenced. The CDR3 sequences were compared.
8.4% = SEQ ID NO: 68
6.1%= SEQ ID NO: 67
2.3% = SEQ ID NO: 62
2% = SEQ ID NO: 60
1.6% = SEQ ID NO:61
1.6% = SEQ ID NO:63
1.3% = SEQ ID NO: 64
1.3% = SEQ ID NO: 66
1.3% = SEQ ID NO: 59

All these VH CDR3 sequences were obtained from libraries V02, V03 and V04, non of the clones above were present in library V01 (colonised patient). The CDR3 (SEQ ID NO: 68) was also present in <1% of library M04 and SEQ ID NO: 59 was also present in <1% of library P01. Six CDR3 sequences are present in at ≥0.9% in more than one library,

### MRSA Libraries

The MRSA VH CDR3 sequences are derived from three patients. One patient has three separate libraries (M02, M03 and M04) corresponding to three blood samples taken 1, 11 and 32 days post infection. Three CDR3 sequences show a reduction over time post-infection. SEQ ID NO: 45 drops from 38% to 12%, SEQ ID NO: 46 drops from 23% to 8% and SEQ ID NO: 48 drops from 10% to less than 1%. The VH chains with these CDR3 are expected to be strongly associated with the MRSA infection. Another of the common CDR3 sequences SEQ ID NO: 47 peaks at 11 days at 24% and then drops to 8%, this common antibody may also be expected to be associated with the MRSA infection. All the above antibodies are being tested for reactivity to MRSA antigens. <

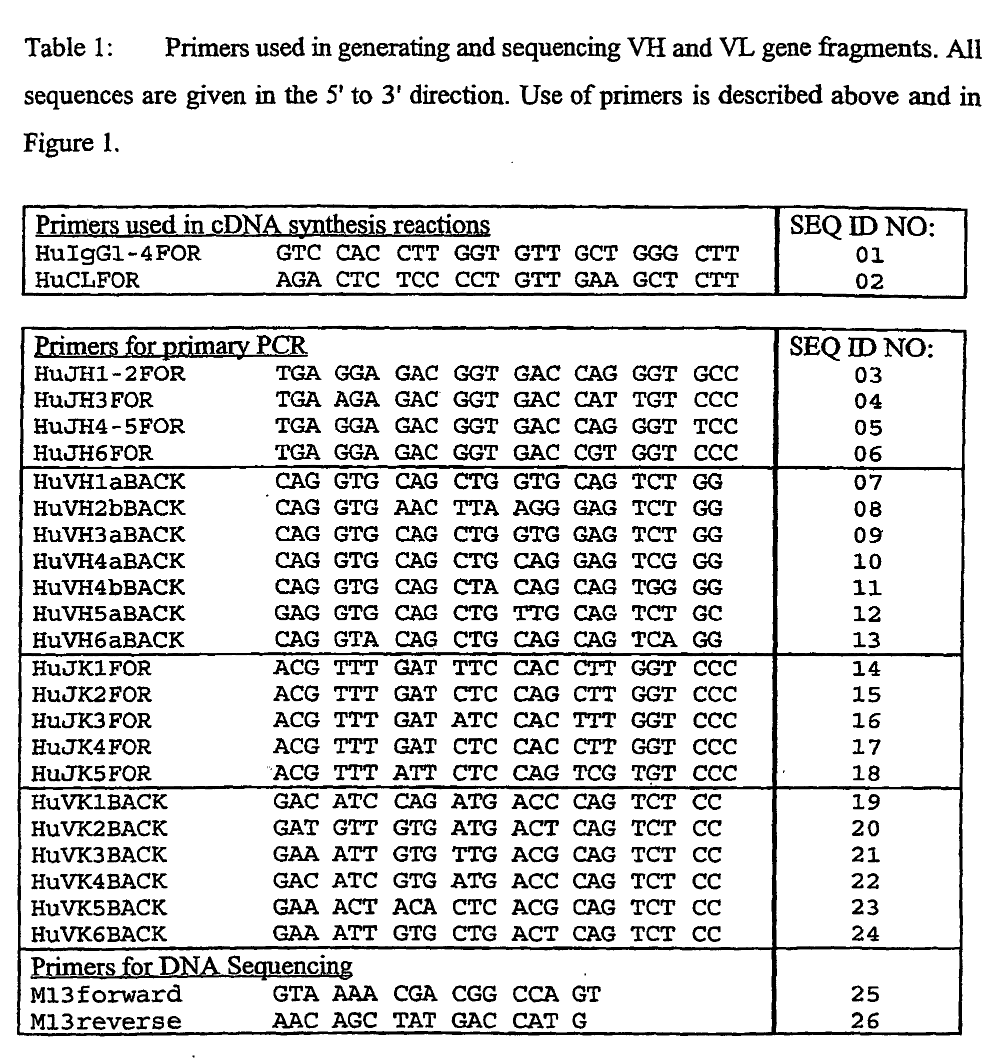

**Table 2:**

| SEQ | V01 | V02 | V03 | V04 | M01 | M02 | M03 | M04 | M05 P01 | |
|---|---|---|---|---|---|---|---|---|---|---|
| 40 | 0 | 0 | 0 | 0 | 13 | 0 | 0 | 0 | 0 | 0 |
| 41 | 0 | 0 | 0 | 0 | 11 | 0 | 0 | 0 | 0 | 0 |
| 42 | 0 | 0 | 0 | 0 | 11 | 0 | 0 | 0 | 0 | 0 |
| 43 | 0 | 0 | 0 | 0 | 5 | 0 | 0 | 0 | 0 | 0 |
| 44 | 0 | 0 | 0 | 0 | 5 | 0 | 0 | 0 | 0 | 0 |
| 45 | 0 | 0 | 0 | 0 | 0 | 38 | 15 | 12 | 0 | 0 |
| 46 0 | 0 | 0 | 0 | 0 | 1 | 23 | 8 | 8 | <1 | 0 |
| 47 | 0 | 0 | 0 | 0 | 1 | 13 | 24 | 8 | 0 | 0 |
| 48 | 0 | 0 | 0 | 0 | 0 | 10 | 1 | <1 | <1 | 0 |
| 49 | 0 | 0 | 0 | 0 | 0 | 3 | 3 | 4 | 0 | 0 |
| 50 | 0 | 0 | 0 | 0 | 1 | 2 | 1 | 5 | 0 | 0 |
| 51 | 0 | 0 | 0 | 0 | 0 | 0 | 0 | 5 | 0 | 0 |
| 52 | 0 | 0 | 0 | 0 | 0 | 0 | 0 | 5 | 0 | 0 |
| 53 | 0 | 0 | 0 | 0 | 0 | 0 | 0 | 0 | 4 | 0 |
| 54 | 0 | 0 | 0 | 0 | 0 | 0 | 0 | 0 | 3 | 0 |
| 55 | 0 | 0 | 0 | 0 | 0 | 0 | 0 | 0 | 3 | 0 |
| 56 | 0 | 0 | 0 | 0 | 0 | 1 | 0 | 1 | 0 | 3 |
| 57 | 0 | 0 | 0 | 0 | 0 | 0 | 0 | 0 | 0 | 3 |
| 58 | 0 | 0 | 0 | 0 | 0 | 0 | 0 | 0 | 0 | 2 |
| 59 | 0 | 0 | 3 | 2 | 0 | 0 | 0 | 0 | 0 | <1 |
| 60 | 0 | 0 | 2 | 9 | 0 | 0 | 0 | 0 | 0 | 0 |
| 61 | 0 | 0 | 2 | 7 | 0 | 0 | 0 | 0 | 0 | 0 |
| 62 | 0 | 9 | 0 | 0 | 0 | 0 | 0 | 0 | 0 | 0 |
| 63 | 0 | 0 | 0 | 12 | 0 | 0 | 0 | 0 | 0 | 0 |
| 64 | 0 | 0 | 4 | 0 | 0 | 0 | 0 | 0 | 0 | 0 |
| 65 | 0 | 5 | 0 | 0 | 0 | 0 | 0 | 0 | 0 | 0 |
| 66 | 0 | 0 | 2 | 5 | 0 | 0 | 0 | 0 | 0 | 0 |
| 67 | 0 | 0 | 1 | 42 | 0 | 0 | 0 | 0 | 0 | 0 |
| 68 | 0 | 0 | 19 | 12 | 0 | 0 | 0 | <1 | 0 | 0 |

## Claims

1. A method for identifying candidate sequences of at least the CDR3 region of antibodies specific against at least one antigen produced by a micro-organism during an infection or against a vaccine, comprising the steps of:
(i) with B cells isolated from at least one patient who has been infected by said micro-organism or administered said vaccine, sequencing at least the CDR3 region of the VH and/or VL coding regions of said B cells; and
(ii) correlating said sequenced at least the CDR3 regions of the VH and VL coding regions of said B cells to identify a set of candidate sequences for at least a CDR3 region of antibodies specific against said at least one antigen produced by said micro-organism or against said vaccine, each of said set of candidate CDR3 sequences or a sequence having at least 60% homology therewith occurring in total at a frequency of at least 1 percent in the set of sequences determined at step (i).

2. A method according to claim 1, said B cells being selected from the group consisting of peripheral B-cell lymphocytes and B cells from the spleen.

3. A method according to claim 2, said peripheral B-cell lymphocytes being isolated from blood from said at least one patient.

4. A method according to claim 1, said at least one antigen being an immunogen.

5. A method according to claim 1, said at least one patient displaying a pronounced antibody response in response to infection by said micro-organism.

6. A method according to claim 1, said at least one patient having recovered from infection by said micro-organism.

7. A method according to claim 1, said correlation step (ii) comprising determining putative amino acid sequences from said sequenced at least the VH and VL CDR3 coding regions, and correlating said putative amino acid sequences.

8. A method according to claim 7, said correlation step (ii) comprising identifying the Complementarity Determining Regions comprised in said at least the VH and VL regions and correlating said Complementarity Determining Regions.

9. A method according to claim 8, said Complementarity Determining Regions being selected from the group consisting of CDR1, CDR2 and CDR3.

10. A method according to claim 1, said correlation step (ii) additionally correlating at least one of the group consisting of: the micro-organism infecting said at least one patient, the site of infection of said at least one patient by said micro-organism, the time point at which said B cells are isolated during infection of said at least one patient by said micro-organism, the age of said at least one patient, the sex of said at least one patient, and the race of said at least one patient.

11. A method according to claim 1, said B cells having been isolated from said at least one patient at a plurality of time points during infection of said at least one patient by said micro-organism, said correlation step (ii) correlating the time point during infection of said at least one patient by said micro-organism at which said B cells are isolated.

12. A method according to claim 1, said B cells having been isolated from at least two patients, at least one of whom has recovered from infection by said micro-organism, and at least one of whom has not recovered from infection by said micro-organism, said correlation step (ii) correlating the recovery of said at least two patients from infection by said micro-organism.

13. A method according to claim 1, said B cells having been isolated from at least two patients, said patients being infected by different micro-organisms producing said at least one antigen, said correlation step (ii) correlating said sequenced at least the VH and VL coding regions of said B cells to identify a set of candidate sequences for antibodies, each of which is specific against at least one shared antigen produced by said different micro-organisms or is specific against different antigens produced by said different micro-organisms.

14. A method of manufacture of a medicament for the treatment of an infection by a micro-organism which produces at least one antigen, comprising the steps of:
(i) performing a method according to any one of the preceding claims; and
(ii) synthesising at least one antibody comprising a candidate sequences specific against said at least one antigen produced by said micro-organism.

15. A method of manufacture of a medicament according to claim 14, comprising the step of mixing said synthesised at least one antibody with a pharmaceutically acceptable carrier, diluant or excipient.

16. A method of producing a database which identifies candidate sequences for antibodies specific against at least one antigen produced by a micro-organism, comprising the steps of:
(i) performing a method according to any one of claims 1-13; and
(ii) storing the data produced by said method in said database.

17. A method of generating a report which identifies candidate sequences for antibodies specific against at least one antigen produced by a micro-organism, comprising the steps of:
(i) performing a method according to any one of claims 1-13; and
(ii) producing a report comprising the data produced by said method.

18. A method for determining the efficacy of a vaccine, comprising the steps of:
(i) with B cells isolated from at least one patient who has been administered said vaccine, sequencing at least the CDR3 region of the VH and/or VL coding regions of said B cells; and
(ii) correlating said sequenced at least the CDR3 region of the VH and/or VL coding regions of said B cells to identify a set of candidate sequences for at least the CDR3 region of antibodies specific against said vaccine, each of said set of CDR3 candidate sequences or a sequence having at least 60% homology therewith occurring in total at a frequency of at least 1 percent in the set of sequences determined at step (i).

19. A method according to claim 18, correlation step (ii) comprising correlating said sequenced at least a CDR3 region of the VH and VL coding regions of said B cells with sequenced at least a CDR3 region of the VH and VL coding regions of B cells isolated from at least one patient who has been infected with a micro-organism against which vaccination with said vaccine is intended to stimulate a protective immune response.

20. A method according to claim 18, said correlation step (ii) additionally correlating at least one of the group consisting of: the time since administration of said vaccine to said at least one patient, the age of said at least one patient, the sex of said at least one patient, the race of said at least one patient, and the Complementarity Determining Regions of said sequenced at least the VH and VL coding regions.

21. A method according to claim 18, said method determining the efficacy of said vaccine in stimulating a protective immune response against the micro-organism against which vaccination with said vaccine is intended to stimulate a protective immune response.

22. A method of producing a database which identifies the efficacy of a vaccine, comprising the steps of:
(i) performing a method according to any one of claims 18-21; and
(ii) storing the data produced by said method in said database.

23. A method of generating a report which identifies the efficacy of a vaccine, comprising the steps of:
(i) performing a method according to any one of claims 18-21; and
(ii) producing a report comprising the data produced by said method.

## Patentansprüche

1. Verfahren zur Identifizierung von Kandidatensequenzen zumindest des CDR3-Bereiches von Antikörpern, die gegenüber mindestens einem Antigen, das durch einen Mikroorganismus während einer Infektion erzeugt wird, oder gegenüber einem Impfstoff spezifisch sind, mit den folgenden Schritten:
(i) Isolierung von B-Zellen mindestens eines Patienten, der durch den Mikroorganismus infiziert wurde oder dem der Impfstoff verabreicht wurde und Sequenzierung zumindest des CDR3-Bereiches der VH und/oder VL codierenden Bereiche der B-Zellen und
(ii) Korrelation der zumindest die CDR3-Bereiche der VH und VL codierenden Bereiche der B-Zellen sequenzierten Zellen, um eine Gruppe von Kandidatensequenzen für wenigstens einen CDR3-Bereich der Antikörper, die gegenüber wenigstens einem Antigen, das durch den Mikroorganismus erzeugt wurde oder gegenüber dem Impfstoff spezifisch sind, zu bestimmen, wobei jede aus der Gruppe von CDR3-Kandidatensequenzen oder einer Sequenz mit wenigstens 60 % Homologie hiermit insgesamt bei einer Frequenz mit wenigstens 1% in der Gruppe von in Schritt (i) bestimmten Sequenzen auftritt.

2. Verfahren nach Anspruch 1, **dadurch gekennzeichnet, dass** die B-Zellen ausgewählt sind aus der Gruppe bestehend aus peripheren B-Zellen-Lymphozyten und B-Zellen der Milz.

3. Verfahren nach Anspruch 2, **dadurch gekennzeichnet, dass** die peripheren B-Zellen-Lymphozyten aus dem Blut von dem mindestens einen Patienten isoliert wurden.

4. Verfahren nach Anspruch 1, **dadurch gekennzeichnet, dass** das wenigstens eine Antigen ein Immunogen ist.

5. Verfahren nach Anspruch 1, **dadurch gekennzeichnet, dass** der mindestens eine Patient eine ausgeprägte Antikörperreaktion in Reaktion auf eine Infektion durch den Mikroorganismus zeigt.

6. Verfahren nach Anspruch 1, **dadurch gekennzeichnet, dass** der mindestens eine Patient von einer Infektion.durch den Mikroorganismus wiederhergestellt ist.

7. Verfahren nach Anspruch 1, **dadurch gekennzeichnet, dass** der Korrelationsschritt (ii) die Bestimmung vermeintlicher Aminosäuresequenzen der mindestens die VH und VL codierenden CDR3-Bereiche sequenzierten Zellen und die Korrelation der vermeintlichen Aminosäurensequenzen aufweist.

8. Verfahren nach Anspruch 7, **dadurch gekennzeichnet, dass** der Korrelationsschritt (ii) die Identifizierung der hypervariablen Bereiche, d.h. CDR-Bereiche, die mindestens in den VH- und VL-Bereichen enthalten sind und die Korrelation dieser hypervariablen Bereiche aufweist.

9. Verfahren nach Anspruch 8, **dadurch gekennzeichnet, dass** die hypervariablen Bereiche ausgewählt sind aus der Gruppe bestehend aus CDR1, CDR2 und CDR3.

10. Verfahren nach Anspruch 1, **dadurch gekennzeichnet, dass** im Korrelationsschritt (ii) zusätzlich wenigstens eine der Gruppen bestehend aus dem den mindestens einen Patienten infizierenden Mikroorganismus, dem Infektionsort des Mikroorganismus bei dem mindestens einen Patienten, dem Zeitpunkt, zu dem die B-Zellen während der Infektion durch den Mikroorganismus des mindestens einen Patienten isoliert wurden, das Alter des mindestens einen Patienten, das Geschlecht des mindestens einen Patienten und die Rasse des mindestens einen Patienten korreliert wird.

11. Verfahren nach Anspruch 1, **dadurch gekennzeichnet, dass** die B-Zellen von mindestens einem Patienten während einer Vielzahl von Zeitpunkten während der Infektion durch den Mikroorganismus beim mindestens einen Patienten von diesem isoliert wurden, wobei der Korrelationsschritt (ii) den Zeitpunkt während der Infektion durch den Mikroorganismus bei dem mindestens einen Patienten, bei dem die B-Zellen isoliert wurden, korreliert wird.

12. Verfahren nach Anspruch 1, **dadurch gekennzeichnet, dass** die B-Zellen von mindestens zwei Patienten isoliert wurden, wobei mindestens einer von diesen sich von einer Infektion durch den Mikroorganismus erholt hat und mindestens einer von ihnen sich von der Infektion mit dem Mikroorganismus nicht erholt hat, wobei der Korrelationsschritt (ii) die Korrelation der Genesung der mindestens zwei Patienten von der Infektion durch den Mikroorganismus aufweist.

13. Verfahren nach Anspruch 1, **dadurch gekennzeichnet, dass** die B-Zellen von mindestens zwei Patienten isoliert wurden, wobei die Patienten durch verschiedene Mikroorganismen, die mindestens ein Antigen erzeugen, infiziert wurden, und der Korrelationsschritt (ii) die Korrelation der mindestens die VH und VL codierenden Bereiche der B-Zellen aufweist, um eine Gruppe von Kandidatensequenzen für Antikörper zu bestimmen, von denen jeder gegenüber mindestens einem geteilten Antigen, der durch verschiedene Mikroorganismen erzeugt wurde, oder gegenüber verschiedenen Antigenen, die durch verschiedene Mikroorganismen erzeugt wurden, spezifisch ist.

14. Verfahren zur Herstellung eines Arzneimittels zur Behandlung einer Infektion durch einen Mikroorganismus, der mindestens ein Antigen erzeugt, mit folgenden Schritten:
(i) Durchführung des Verfahrens nach mindestens einem der vorhergehenden Ansprüche und
(ii) Herstellung mindestens eines Antikörpers, der eine Kandidatensequenz enthält, die gegenüber mindestens einem durch den Mikroorganismus erzeugten Antigen spezifisch ist.

15. Verfahren zur Herstellung eines Arzneimittels nach Anspruch 14, **dadurch gekennzeichnet, dass** zusätzlich der Schritt der Vermischung des mindestens einen Antikörpers mit einem pharmazeutisch verträglichen Carrier, Verdünnungsmittel oder Arzneistoffträger enthalten ist.

16. Verfahren zur Erstellung einer Datenbank, die die Kandidatensequenzen für Antikörper, die gegenüber wenigstens einem durch einen Mikroorganismus erzeugten Antigen spezifisch sind, mit folgenden Schritten:
(i) Durchführung des Verfahrens nach einem der Ansprüche 1 bis 13, und
(ii) Speicherung der durch das Verfahren erstellten Daten in der Datenbank.

17. Verfahren zur Erstellung eines Reports, der die Kandidatensequenzen für Antikörper, die gegenüber einem durch einen Mikroorganismus erzeugten Antigen spezifisch sind, mit folgenden Schritten:
(i) Durchführung des Verfahrens nach einem der Ansprüche 1 bis 13, und
(ii) Erstellung eines Reports, der die durch das Verfahren erstellten Daten aufweist.

18. Verfahren zur Bestimmung der Wirksamkeit eines Impfstoffes mit folgenden Schritten:
(i) Isolierung von B-Zellen von mindestens einem Patienten, dem der Impfstoff verabreicht wurde, wobei wenigstens der CDR3-Bereich der VH und/oder VL codierenden Bereiche der B-Zellen sequenziert werden, und
(ii) Korrelation der mindestens den CDR3-Bereich, der VH und/oder VL codierenden Bereiche der B-Zellen sequenzierten Zellen, um eine Gruppe von Kandidatensequenzen für mindestens den CDR3-Bereich von gegenüber dem Impfstoff spezifischen Antikörpern zu identifizieren, wobei jede aus der Gruppe der CDR3-Kandidatense-quenzen oder einer Sequenz mit einer Homologie von mindestens 60 % hiervon insgesamt mit einer Frequenz von mindestens 1% der Gruppe in der in Schritt (i) bestimmten Sequenzen auftritt.

19. Verfahren nach Anspruch 18, wobei der Korrelationsschritt (ii) die Korrelation der mindestens einen CDR3-Bereich der VH und VL codierenden Bereiche der B-Zellen sequenzierten Zellen mit mindestens einem CDR3-Bereich der VH und VL codierenden Bereiche der B-Zellen sequenzierten Zellen, die von mindestens einem Patienten isoliert wurden, der mit einem Mikroorganismus, gegen den eine Impfung mit dem Impfstoff zur Stimulation einer schützenden Immunantwort beabsichtigt ist, aufweist.

20. Verfahren nach Anspruch 18, **dadurch gekennzeichnet, dass** der Korrelationsschritt (ii) zusätzlich die Korrelation von Daten aus der Gruppe bestehend aus der seit der dem mindestens einen Patienten erfolgten Verabreichung des Impfstoffs vergangenen Zeit, dem Alter des mindestens einen Patienten, dem Geschlecht des mindestens einen Patienten, der Rasse des mindestens einen Patienten und den hypervariablen Bereichen, d.h. CDR-Bereichen, der mindestens die VH und VL codierenden Bereiche sequenzierten Zellen.

21. Verfahren nach Anspruch 18, **dadurch gekennzeichnet, dass** das Verfahren die Wirksamkeit des Impfstoffs zur Stimulierung einer schützenden Immunantwort gegenüber dem Mikroorganismus, gegen den die Impfung mit dem Impfstoff zur Stimulierung einer schützenden Immunantwort beabsichtigt ist, bestimmt wird.

22. Verfahren zur Erstellung einer Datenbank, die die Wirksamkeit eines Impfstoffs identifiziert, mit folgenden Schritten:
(i) Durchführung eines Verfahrens nach einem der Ansprüche 18 bis 21, und
(ii) Speicherung der durch das Verfahren erstellten Daten in der Datenbank.

23. Verfahren zur Erstellung eines Reports, der die Wirksamkeit eines Impfstoffes identifiziert mit folgenden Schritten:
(i) Durchführung eines Verfahrens nach einem der Ansprüche 18 bis 21 und
(ii) Erstellung eines Reports, der die durch das Verfahren erstellten Daten enthält.

## Revendications

1. Procédé pour identifier des séquences candidates d'au moins la région CDR3 d'anticorps spécifiques contre au moins un antigène produit par un micro-organisme pendant une infection ou contre un vaccin, comprenant les étapes de :
(i) avec des cellules B isolées auprès d'un patient qui a été infecté par ledit micro-organisme ou auquel ledit vaccin a été administré, séquençage au moins de la région CDR3 des régions codant VH et/ou VL desdites cellules B ; et
(ii) corrélation au moins de ladite région CDR3 séquencée des régions codant VH et VL desdites cellules B pour identifier un ensemble de séquences candidates pour au moins une région CDR3 d'anticorps spécifiques contre ledit ou lesdits antigènes produits par ledit micro-organisme ou contre ledit vaccin, chaque séquence dudit ensemble de séquences CDR3 candidates ou une séquence ayant au moins 60 % d'homologie avec elle survenant au total à une fréquence d'au moins 1 % dans l'ensemble de séquences déterminées à l'étape (i).

2. Procédé selon la revendication 1, lesdites cellules B étant choisies dans le groupe consistant en les lymphocytes B périphériques et les cellules B provenant de la rate.

3. Procédé selon la revendication 2, lesdits lymphocytes B périphériques étant isolés à partir du sang dudit ou desdits patients.

4. Procédé selon la revendication 1, ledit ou lesdits antigènes étant un immunogène.

5. Procédé selon la revendication 1, ledit ou lesdits patients présentant une réponse anticorps prononcée en réponse à une infection par ledit micro-organisme.

6. Procédé selon la revendication 1, ledit ou lesdits patients s'étant rétablis à la suite d'une infection par ledit micro-organisme.

7. Procédé selon la revendication 1, ladite étape de corrélation (ii) comprenant la détermination de séquences d'aminoacides supposées d'après au moins lesdites régions codant CDR3 de VH et VL et la corrélation desdites séquences d'aminoacides supposées.

8. Procédé selon la revendication 7, ladite étape de corrélation (ii) comprenant l'identification des régions déterminant la complémentarité comprises dans au moins lesdites régions VH et VL et la corrélation desdites régions déterminant la complémentarité.

9. Procédé selon la revendication 8, lesdites régions déterminant la complémentarité étant choisies dans le groupe consistant en CDR1, CDR2 et CDR3.

10. Procédé selon la revendication 1, ladite étape de corrélation (ii) corrélant en outre au moins un membre du groupe consistant en : le micro-organisme infectant ledit ou lesdits patients, le site d'infection dudit ou desdits patients par ledit micro-organisme, l'instant auquel lesdites cellules B sont isolées pendant l'infection dudit ou desdits patients par ledit micro-organisme, l'âge dudit ou desdits patients, le sexe dudit ou desdits patients et la race dudit ou desdits patients.

11. Procédé selon la revendication 1, lesdites cellules B ayant été isolées auprès dudit ou desdits patients à une pluralité d'instants pendant l'infection dudit ou desdits patients par ledit micro-organisme, ladite étape de corrélation (ii) corrélant l'instant pendant l'infection dudit ou desdits patients par ledit micro-organisme auquel lesdites cellules B sont isolées.

12. Procédé selon la revendication 1, lesdites cellules Bayant été isolées auprès d'au moins deux patients, dont l'un au moins s'est rétabli à la suite d'une infection par ledit micro-organisme, et dont l'un au moins ne s'est pas rétabli à la suite d'une infection par ledit micro-organisme, ladite étape de corrélation (ii) corrélant le rétablissement desdits au moins deux patients à la suite d'une infection par ledit micro-organisme.

13. Procédé selon la revendication 1, lesdites cellules B ayant été isolées auprès d'au moins deux patients, lesdits patients étant infectés par des micro-organismes différents produisant ledit ou lesdits antigènes, ladite étape de corrélation (ii) corrélant au moins les régions codant VH et VL séquencées desdites cellules B pour identifier un ensemble de séquences candidates pour des anticorps qui sont spécifiques chacun contre au moins un antigène partagé produit par lesdits micro-organismes différents ou spécifiques chacun contre des antigènes différents produits par lesdits micro-organismes différents.

14. Procédé de production d'un médicament pour le traitement d'une infection par un micro-organisme qui produit au moins un antigène, comprenant les étapes de :
(i) mise en oeuvre d'un procédé selon l'une quelconque des revendications précédentes ; et
(ii) synthèse d'au moins un anticorps comprenant des séquences candidates spécifiques contre ledit ou lesdits antigènes produits par ledit micro-organisme.

15. Procédé de production d'un médicament selon la revendication 14 comprenant l'étape de mélange dudit ou desdits anticorps synthétisés avec un support, diluant ou excipient pharmaceutiquement acceptable.

16. Procédé de production d'une base de données qui identifie des séquences candidates pour des anticorps spécifiques contre au moins un antigène produit par un micro-organisme, comprenant les étapes de :
(i) mise en oeuvre d'un procédé selon l'une quelconque des revendications 1-13 ; et
(ii) stockage des données produites par ledit procédé dans ladite base de données.

17. Procédé de production d'un rapport qui identifie des séquences candidates pour des anticorps spécifiques contre au moins un antigène produit par un micro-organisme, comprenant les étapes de :
(i) mise en oeuvre d'un procédé selon l'une quelconque des revendications 1-13 ; et
(ii) production d'un rapport comprenant les données produites par ledit procédé.

18. Procédé pour déterminer l'efficacité d'un vaccin, comprenant les étapes de :
(i) avec des cellules B isolées auprès d'au moins un patient auquel a été administré ledit vaccin, séquençage d'au moins la région CDR3 des régions codant VH et/ou VL desdites cellules B ; et
(ii) corrélation au moins de ladite région CDR3 séquencée des régions codant VH et/ou VL desdites cellules B pour identifier un ensemble de séquences candidates pour au moins la région CDR3 d'anticorps spécifiques contre ledit vaccin, chaque séquence dudit ensemble de séquences candidates de CDR3 ou une séquence ayant au moins 60 % d'homologie avec elle survenant au total à une fréquence d'au moins 1 % dans l'ensemble de séquences déterminé à l'étape (i).

19. Procédé selon la revendication 18, l'étape de corrélation (ii) comprenant la corrélation au moins de ladite région CDR3 séquencée des régions codant VH et VL desdites cellules B avec au moins une région CDR3 séquencée des régions codant VH et VL de cellules B isolées auprès d'au moins un patient qui a été infecté avec un micro-organisme contre lequel une vaccination avec ledit vaccin est envisagée pour stimuler une réponse immunitaire protectrice.

20. Procédé selon la revendication 18, ladite étape de corrélation (ii) corrélant en outre au moins un membre du groupe consistant en : la durée depuis l'administration dudit vaccin audit patient ou auxdits patients, l'âge dudit ou desdits patients, le sexe dudit ou desdits patients, la race dudit ou desdits patients, et les régions déterminant la complémentarité au moins desdites régions codant VH et VL séquencées.

21. Procédé selon la revendication 18, ledit procédé déterminant l'efficacité dudit vaccin dans la stimulation d'une réponse immunitaire protectrice contre le micro-organisme contre lequel une vaccination avec ledit vaccin est envisagée pour stimuler une réponse immunitaire protectrice.

22. Procédé de production d'une base de données qui identifie l'efficacité d'un vaccin, comprenant les étapes de :
(i) mise en oeuvre d'un procédé selon l'une quelconque des revendications 18-21 ; et
(ii) stockage des données produites par ledit procédé dans ladite base de données.

23. Procédé de production d'un rapport qui identifie l'efficacité d'un vaccin, comprenant les étapes de :
(i) mise en oeuvre d'un procédé selon l'une quelconque des revendications 18-21 ; et
(ii) production d'un rapport comprenant les données produites par ledit procédé.
